# EUROPEAN PATENT SPECIFICATION

(11) **EP 3 986 346 B1**
(45) Date of publication and mention of the grant of the patent: **14.02.2024**
(21) Application number: 20729342.4
(22) Date of filing: 05.05.2020
(51) Int. Cl.: A61F 13/00

(54) **PEEL AND PLACE DRESSING FOR NEGATIVE-PRESSURE THERAPY**
PEEL-AND-PLACE-VERBAND FÜR UNTERDRUCKTHERAPIE
PANSEMENT À DÉCOLLER ET PLACER POUR THÉRAPIE PAR PRESSION NÉGATIVE

(30) Priority: 20.06.2019 US 201962864185 P
(43) Date of publication of application: 27.04.2022
(73) Proprietor: 3M Innovative Properties Company, St. Paul, MN 55133-3427 (US)
(72) Inventor: LONG, Justin Alexander, San Antonio, TX 78265 (US); LOCKE, Christopher Brian, San Antonio, TX 78265 (US); ROBINSON, Timothy Mark, San Antonio, TX 78265 (US)
(74) Representative: Simmons & Simmons
(86) International application number: PCT/US2020/031510
(87) International publication number: WO 2020/256843

(56) References cited:
- CA-A1- 3 061 327
- US-A1- 2018 353 339

## Description

### TECHNICAL FIELD

The invention set forth in the appended claims relates generally to tissue treatment systems and more particularly, but without limitation, to dressings for tissue treatment.

### BACKGROUND

Clinical studies and practice have shown that reducing pressure in proximity to a tissue site can augment and accelerate growth of new tissue at the tissue site. The applications of this phenomenon are numerous, but it has proven particularly advantageous for treating wounds. Regardless of the etiology of a wound, whether trauma, surgery, or another cause, proper care of the wound is important to the outcome. Treatment of wounds or other tissue with reduced pressure may be commonly referred to as "negative-pressure therapy," but is also known by other names, including "negative-pressure wound therapy," "reduced-pressure therapy," "vacuum therapy," "vacuum-assisted closure," and "topical negative-pressure," for example. Negative-pressure therapy may provide a number of benefits, including migration of epithelial and subcutaneous tissues, improved blood flow, and micro-deformation of tissue at a wound site. Together, these benefits can increase development of granulation tissue and reduce healing times.

There is also widespread acceptance that cleansing a tissue site can be highly beneficial for new tissue growth. For example, a wound or a cavity can be washed out with a liquid solution for therapeutic purposes. These practices are commonly referred to as "irrigation" and "lavage" respectively. "Instillation" is another practice that generally refers to a process of slowly introducing fluid to a tissue site and leaving the fluid for a prescribed period of time before removing the fluid. For example, instillation of topical treatment solutions over a wound bed can be combined with negative-pressure therapy to further promote wound healing by loosening soluble contaminants in a wound bed and removing infectious material. As a result, soluble bacterial burden can be decreased, contaminants removed, and the wound cleansed.

In this regard, US 2018/0353339 A1 concerns composite dressings for improved granulation and reduced maceration with negative-pressure treatment.

CA 3061327 concerns a peel and place dressing for negative-pressure therapy.

### BRIEF SUMMARY

The present invention provides a dressing according to claim 1. Optional features are set out in the dependent claims.

Illustrative embodiments are also provided to enable a person skilled in the art to make and use the claimed subject matter.

In some examples, the sealing layer may comprise or consist essentially of a layer of perforated gel, such as a silicone gel. A central area of the gel may be removed or perforated to define a treatment area. The fluid control layer may comprise or consist essentially of a polyurethane film having fluid restrictions, such as fenestrations in the film. The film may be backed with an acrylic adhesive in some embodiments. The manifold may be a reticulated foam in some examples, and the polyurethane film may be laminated to the manifold or the acrylic adhesive on the polyurethane film can bond the two together. In some examples, the polyurethane film may be laminated to the manifold and then cut to a desired size and shape, which can simplify manufacturing processes.

In some embodiments, the manifold and the fluid control layer may have a diameter that is larger than the treatment aperture, so that the edge of the manifold is not exposed when assembled and applied to a tissue site. The fluid control layer may be disposed over the treatment aperture so that a substantial number of the fluid restrictions are aligned with the treatment aperture. For example, the manifold and fluid control layer may be substantially aligned with the treatment aperture, although a wide tolerance may be acceptable. The manifold and the fluid control layer may overlay an area of the sealing layer around the treatment aperture, and the sealing layer may have an adhesive in the overlay area to secure the manifold, the fluid control layer, or both. The cover may be positioned over the assembled manifold and fluid control layer and adhered to the sealing layer to enclose the manifold.

In some embodiments, the sealing layer may comprise or consist essentially of a layer of perforated gel, such as a silicone gel, having perforations continuously distributed across the sealing layer. Fluid restrictions in the fluid control layer may be disposed within the perforations, which can provide similar functionality to the treatment aperture while increasing the surface area of the sealing layer.

More generally, a dressing for treating a tissue site with negative pressure may comprise a sealing layer having a treatment aperture and a plurality of perforations around the treatment aperture, and a fluid control layer having a plurality of fluid restrictions aligned with the treatment aperture. A manifold may be disposed adjacent to the fluid restrictions, and a cover comprising a non-porous film may be disposed over the manifold and coupled to the sealing layer around the manifold. The cover may additionally have a pressure-sensitive adhesive disposed adjacent to the plurality of perforations. In more particular embodiments, the fluid control layer may comprise or consist essentially of a polyurethane film. The sealing layer may be formed from a gel, such as a silicone gel in some embodiments.

In some examples, the manifold may have a first edge defining a manifold face adjacent to the fluid control layer, and the fluid control layer may have a second edge defining a fluid control face adjacent to the manifold face. The fluid control face and the manifold face may have a similar shape in some embodiments. The manifold face may be at least as large as the fluid control face, and the fluid control face may be larger than the treatment aperture. In more specific examples, at least one of the manifold and the fluid control layer may be coupled to a margin around the treatment aperture.

Alternatively, other example embodiments of a dressing for treating a tissue site with negative pressure may comprise a manifold and a fluid control layer comprising a plurality of fluid restrictions adjacent to the manifold. A sealing layer comprising a plurality of perforations may be disposed adjacent to the fluid control layer and at least some of the perforations can be aligned with more than one of the fluid restrictions. A cover comprising a non-porous film may be disposed over the manifold and coupled to the sealing layer around the manifold. The cover may additionally have a pressure-sensitive adhesive disposed adjacent to the plurality of perforations. In more particular embodiments, the fluid control layer may comprise or consist essentially of a polyurethane film. The sealing layer may be formed from a gel, such as a silicone gel in some embodiments.

In more particular examples, the fluid restrictions may comprise slits, which may have a length of about 2 millimeters to about 5 millimeters. Apertures in the sealing layer may be circular, having a diameter sufficiently large to align with more than one of the fluid restrictions. For example, a diameter in a range of about 7 millimeters to about 9 millimeters may be suitable for some configurations.

In some embodiments, a dressing for treating a tissue site with negative pressure may comprise a cover having an adhesive, a manifold, a perforated polymer film, and a perforated silicone gel having a treatment aperture. The cover, the manifold, the perforated polymer film, and the perforated silicone gel may be assembled in a stacked relationship with the cover and the perforated silicone gel enclosing the manifold. The perforated polymer film may be at least partially exposed through the treatment aperture, and at least some of the adhesive may be exposed through the perforated silicone around the treatment aperture.

A dressing for treating a tissue site with negative pressure may comprise a manifold, a gel layer, a fluid control layer, and a cover in some embodiments. The gel layer may comprise an open central window and a plurality of openings around the open central window. The fluid control layer may extend across the open central window and comprise a plurality of fluid restrictions. The cover may comprise a non-porous film and a pressure-sensitive adhesive, and the non-porous film may be disposed over the manifold and coupled to the gel layer around the manifold, and the pressure-sensitive adhesive may be disposed adjacent to the plurality of perforations.

In some embodiments, a dressing for treating a tissue site with negative pressure may comprise a foam manifold for the passage of negative pressure and passage of wound fluid; a lower surface having an open area for delivery of negative pressure and passage of wound fluid via the manifold, the open area being surrounded by a drape area for sealing to tissue, the drape area having an adhesive and not including openings for the passage of negative pressure via the manifold; and a polymer film wound contact layer extending across the open area in the lower surface and having openings for the passage of negative pressure and wound fluid into the foam manifold. The dressing may further comprise a cover in some embodiments, the cover comprising a drape disposed over the manifold and coupled to the drape area around the manifold.

In further embodiments, a dressing is also provided for contacting a tissue site comprising a tissue contact layer having a tissue facing side and an environment facing side. The tissue contact layer may comprises one or more of a perforated layer having a tissue facing side and an environment facing side, the perforated layer having a plurality of perforations; and/or a fluid control layer having a tissue facing side and an environment facing side, the fluid control layer having a plurality of fluid restrictions. In addition to the tissue contact layer, the dressing may also include a porous layer capable of distributing a negative pressure and/or an instillation fluid to the tissue site. The porous layer may be located on the environment facing side of the tissue contact layer. A drape capable of sealing the tissue site for distribution of the negative pressure and/or instillation fluid may also be included. The drape can form an aperture capable of receiving a negative pressure and/or instillation fluid and be located on the environment facing side of the porous layer. The perforated layer and/or the fluid control layer may comprise citric acid, and/or citric acid may be present on one or more surfaces of the perforated layer and/or one or more surfaces of the fluid control layer.

Also disclosed herein is a method of treating a tissue site with negative pressure. The method may comprise applying the dressing to a tissue site, sealing the dressing to epidermis adjacent to the tissue site, fluidly coupling the dressing to a negative-pressure source, and applying negative pressure from the negative-pressure source. In some embodiments, applying the dressing may comprise disposing at least part of the dressing across an edge of the tissue site.

A first layer of the dressing may not be substantially exposed to the tissue site during the step of applying negative pressure. Further, at least one layer of the dressing can be configured to be exposed to the tissue site during the step of applying negative pressure. Additionally, applying negative pressure may open the fluid restrictions.

The method may also comprise reducing negative pressure from the negative-pressure source, wherein reducing negative pressure closes the fluid restrictions.

At least one of the sealing layer and the fluid control layer may be at least partially comprised between the manifold and the tissue site during the step of applying negative pressure.

The entire manifold may be comprised on the opposite side of at least one of the sealing layer and the fluid control layer in relation to the tissue site during the step of applying negative pressure.

Applying the dressing may comprise disposing at least part of the dressing across an edge of the tissue site.

The method may also comprise fluidly coupling a fluid container between the dressing and the negative-pressure source, and transferring exudate from the dressing to the fluid container.

The method may also comprise applying a manifold between the dressing and the tissue site.

Additionally, methods of making the dressings are disclosed herein. The method may comprise adding citric acid to a polymer solution used to form the sealing layer and/or the fluid control layer prior to casting the sealing layer and/or the fluid control layer; and/or applying a citric acid-containing polymer solution to one or more surfaces of the sealing layer and/or one or more surfaces of the fluid control layer to form a coating comprising citric acid on the one or more surfaces of the sealing layer and/or the fluid control layer.

A kit is also disclosed herein that contains components of any of the dressings disclosed herein. The kit may further contain components for use in a negative pressure wound therapy, such as any one or more of a fluid container, a fluid conduit configured to deliver negative pressure to the dressing, and/or a negative pressure source.

Objectives, advantages, and a preferred mode of making and using the claimed subject matter may be understood best by reference to the accompanying drawings in conjunction with the following detailed description of illustrative embodiments.

### BRIEF DESCRIPTION OF THE DRAWINGS

Figure 1 is a functional block diagram of an example embodiment of a therapy system that can provide negative-pressure treatment and instillation treatment in accordance with this specification;
Figure 2A is an assembly view of an example of a dressing, illustrating additional details that may be associated with some example embodiments of the therapy system of Figure 1;
Figure 2B is another assembly view of an example of a dressing, illustrating additional details that may be associated with some example embodiments of the therapy system of Figure 1;
Figure 3 is a top view of the example dressing of Figure 2A;
Figure 4 is a bottom view of the example dressing of Figure 2A;
Figure 5 is an assembly view of another example of a dressing, illustrating additional details that may be associated with some example embodiment of the therapy system of Figure 1;
Figure 6 is a schematic view of an example configuration of fluid restrictions in a layer that may be associated with some embodiments of the dressing of Figure 2A or Figure 5;
Figure 7 is a schematic view of an example configuration of apertures in a layer that may be associated with some embodiments of the dressing of Figure 5; and
Figure 8 is a schematic view of the example layer of Figure 6 overlaid on the example layer of Figure 7.

### DESCRIPTION OF EXAMPLE EMBODIMENTS

The following description of example embodiments provides information that enables a person skilled in the art to make and use the subject matter set forth in the appended claims, but it may omit certain details already well-known in the art. The following detailed description is, therefore, to be taken as illustrative and not limiting.

The example embodiments may also be described herein with reference to spatial relationships between various elements or to the spatial orientation of various elements depicted in the attached drawings. In general, such relationships or orientation assume a frame of reference consistent with or relative to a patient in a position to receive treatment. However, as should be recognized by those skilled in the art, this frame of reference is merely a descriptive expedient rather than a strict prescription.

### Example Therapy System

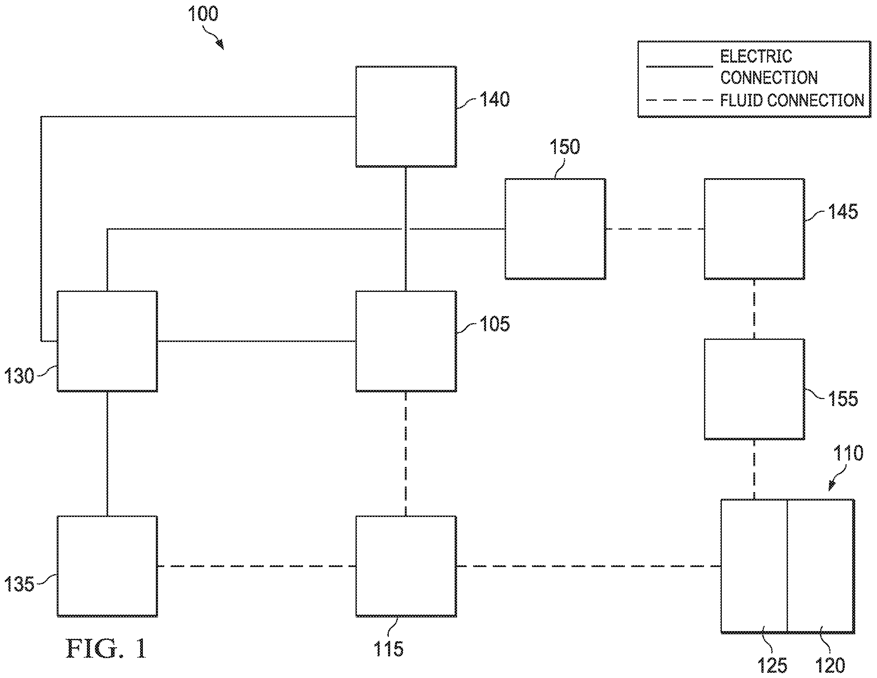

Figure 1 is a simplified functional block diagram of an example embodiment of a therapy system 100 that can provide negative-pressure therapy with instillation of topical treatment solutions to a tissue site in accordance with this specification.

The term "tissue site" in this context broadly refers to a wound, defect, or other treatment target located on or within tissue, including, but not limited to, bone tissue, adipose tissue, muscle tissue, neural tissue, dermal tissue, vascular tissue, connective tissue, cartilage, tendons, or ligaments. A wound may include chronic, acute, traumatic, subacute, and dehisced wounds, partial-thickness burns, ulcers (such as diabetic, pressure, or venous insufficiency ulcers), flaps, and grafts, for example. The term "tissue site" may also refer to areas of any tissue that are not necessarily wounded or defective but are instead areas in which it may be desirable to add or promote the growth of additional tissue. For example, negative pressure may be applied to a tissue site to grow additional tissue that may be harvested and transplanted.

The therapy system 100 may include a source or supply of negative pressure, such as a negative-pressure source 105, and one or more distribution components. A distribution component is preferably detachable and may be disposable, reusable, or recyclable. A dressing, such as a dressing 110, and a fluid container, such as a container 115, are examples of distribution components that may be associated with some examples of the therapy system 100. As illustrated in the example of Figure 1, the dressing 110 may comprise or consist essentially of a tissue interface 120, a cover 125, or both in some embodiments.

A fluid conductor is another illustrative example of a distribution component. A "fluid conductor," in this context, broadly includes a tube, pipe, hose, conduit, or other structure with one or more lumina or open pathways adapted to convey a fluid between two ends. Typically, a tube is an elongated, cylindrical structure with some flexibility, but the geometry and rigidity may vary. Moreover, some fluid conductors may be molded into or otherwise integrally combined with other components. Distribution components may also include or comprise interfaces or fluid ports to facilitate coupling and de-coupling other components. In some embodiments, for example, a dressing interface may facilitate coupling a fluid conductor to the dressing 110. For example, such a dressing interface may be a SENSAT.R.A.C.^{™} Pad available from Kinetic Concepts, Inc. of San Antonio, Texas.

The therapy system 100 may also include a regulator or controller, such as a controller 130. Additionally, the therapy system 100 may include sensors to measure operating parameters and provide feedback signals to the controller 130 indicative of the operating parameters. As illustrated in Figure 1, for example, the therapy system 100 may include a first sensor 135 and a second sensor 140 coupled to the controller 130.

The therapy system 100 may also include a source of instillation solution. For example, a solution source 145 may be fluidly coupled to the dressing 110, as illustrated in the example embodiment of Figure 1. The solution source 145 may also be a container, canister, pouch, bag, or other storage component, which can provide a solution for instillation therapy. Compositions of solutions may vary according to a prescribed therapy, but examples of solutions that may be suitable for some prescriptions include hypochlorite-based solutions, silver nitrate (0.5%), sulfur-based solutions, biguanides, cationic solutions, and isotonic solutions.

The solution source 145 may be fluidly coupled to a positive-pressure source such as a positive-pressure source 150, a negative-pressure source such as the negative-pressure source 105, or both in some embodiments. A regulator, such as an instillation regulator 155, may also be fluidly coupled to the solution source 145 and the dressing 110 to ensure proper dosage of instillation solution (e.g. saline) to a tissue site. For example, the instillation regulator 155 may comprise a piston that can be pneumatically actuated by the negative-pressure source 105 to draw instillation solution from the solution source during a negative-pressure interval and to instill the solution to a dressing during a venting interval. Additionally or alternatively, the controller 130 may be coupled to the negative-pressure source 105, the positive-pressure source 150, or both, to control dosage of instillation solution to a tissue site. In some embodiments, the instillation regulator 155 may also be fluidly coupled to the negative-pressure source 105 through the dressing 110, as illustrated in the example of Figure 1.

Some components of the therapy system 100 may be housed within or used in conjunction with other components, such as sensors, processing units, alarm indicators, memory, databases, software, display devices, or user interfaces that further facilitate therapy. For example, in some embodiments, the negative-pressure source 105 may be combined with the controller 130, the solution source 145, and other components into a therapy unit.

In general, components of the therapy system 100 may be coupled directly or indirectly. For example, the negative-pressure source 105 may be directly coupled to the container 115 and may be indirectly coupled to the dressing 110 through the container 115. Coupling may include fluid, mechanical, thermal, electrical, or chemical coupling (such as a chemical bond), or some combination of coupling in some contexts. For example, the negative-pressure source 105 may be electrically coupled to the controller 130 and may be fluidly coupled to one or more distribution components to provide a fluid path to a tissue site. In some embodiments, components may also be coupled by virtue of physical proximity, being integral to a single structure, or being formed from the same piece of material.

A negative-pressure supply, such as the negative-pressure source 105, may be a reservoir of air at a negative pressure or may be a manual or electrically-powered device, such as a vacuum pump, a suction pump, a wall suction port available at many healthcare facilities, or a micro-pump, for example. "Negative pressure" generally refers to a pressure less than a local ambient pressure, such as the ambient pressure in a local environment external to a sealed therapeutic environment. In many cases, the local ambient pressure may also be the atmospheric pressure at which a tissue site is located. Alternatively, the pressure may be less than a hydrostatic pressure associated with tissue at the tissue site. Unless otherwise indicated, values of pressure stated herein are gauge pressures. References to increases in negative pressure typically refer to a decrease in absolute pressure, while decreases in negative pressure typically refer to an increase in absolute pressure. While the amount and nature of negative pressure provided by the negative-pressure source 105 may vary according to therapeutic requirements, the pressure is generally a low vacuum, also commonly referred to as a rough vacuum, between -5 mm Hg (-667 Pa) and -500 mm Hg (-66.7 kPa). Common therapeutic ranges are between -50 mm Hg (-6.7 kPa) and -300 mm Hg (-39.9 kPa).

The container 115 is representative of a container, canister, pouch, or other storage component, which can be used to manage exudates and other fluids withdrawn from a tissue site. In many environments, a rigid container may be preferred or required for collecting, storing, and disposing of fluids. In other environments, fluids may be properly disposed of without rigid container storage, and a re-usable container could reduce waste and costs associated with negative-pressure therapy.

A controller, such as the controller 130, may be a microprocessor or computer programmed to operate one or more components of the therapy system 100, such as the negative-pressure source 105. In some embodiments, for example, the controller 130 may be a microcontroller, which generally comprises an integrated circuit containing a processor core and a memory programmed to directly or indirectly control one or more operating parameters of the therapy system 100. Operating parameters may include the power applied to the negative-pressure source 105, the pressure generated by the negative-pressure source 105, or the pressure distributed to the tissue interface 120, for example. The controller 130 is also preferably configured to receive one or more input signals, such as a feedback signal, and programmed to modify one or more operating parameters based on the input signals.

Sensors, such as the first sensor 135 and the second sensor 140, are generally known in the art as any apparatus operable to detect or measure a physical phenomenon or property, and generally provide a signal indicative of the phenomenon or property that is detected or measured. For example, the first sensor 135 and the second sensor 140 may be configured to measure one or more operating parameters of the therapy system 100. In some embodiments, the first sensor 135 may be a transducer configured to measure pressure in a pneumatic pathway and convert the measurement to a signal indicative of the pressure measured. In some embodiments, for example, the first sensor 135 may be a piezo-resistive strain gauge. The second sensor 140 may optionally measure operating parameters of the negative-pressure source 105, such as a voltage or current, in some embodiments. Preferably, the signals from the first sensor 135 and the second sensor 140 are suitable as an input signal to the controller 130, but some signal conditioning may be appropriate in some embodiments. For example, the signal may need to be filtered or amplified before it can be processed by the controller 130. Typically, the signal is an electrical signal, but may be represented in other forms, such as an optical signal.

In operation, the tissue interface 120 may be placed within, over, on, or otherwise proximate to a tissue site. If the tissue site is a wound, for example, the tissue interface 120 may partially or completely fill the wound, or it may be placed over the wound. The cover 125 may be placed over the tissue interface 120 and sealed to an attachment surface near a tissue site. For example, the cover 125 may be sealed to undamaged epidermis peripheral to a tissue site. Thus, the dressing 110 can provide a sealed therapeutic environment proximate to a tissue site, substantially isolated from the external environment, and the negative-pressure source 105 can reduce pressure in the sealed therapeutic environment.

### Example Dressings

The tissue interface 120 can be generally adapted to partially or fully contact a tissue site. The tissue interface 120 may take many forms, and may have many sizes, shapes, or thicknesses, depending on a variety of factors, such as the type of treatment being implemented or the nature and size of a tissue site. For example, the size and shape of the tissue interface 120 may be adapted to the contours of deep and irregular shaped tissue sites. Any or all of the surfaces of the tissue interface 120 may have an uneven, coarse, or jagged profile.

In some embodiments, the tissue interface 120 may comprise or consist essentially of a manifold. A manifold in this context may comprise or consist essentially of a means for collecting or distributing fluid across the tissue interface 120 under pressure and/or under reduced pressure. For example, a manifold may be adapted to receive negative pressure from a source and distribute negative pressure through multiple apertures across the tissue interface 120, which may have the effect of collecting fluid from across a tissue site and drawing the fluid toward the source. In some embodiments, the fluid path may be reversed, or a secondary fluid path may be provided to facilitate delivering fluid, such as fluid from a source of instillation solution, across a tissue site.

In some embodiments, the cover 125 may provide a bacterial barrier and protection from physical trauma. The cover 125 may also be constructed from a material that can reduce evaporative losses and provide a fluid seal between two components or two environments, such as between a therapeutic environment and a local external environment. The cover 125 may comprise or consist of, for example, an elastomeric film or membrane that can provide a seal adequate to maintain a negative pressure at a tissue site for a given negative-pressure source. The cover 125 may have a high moisture-vapor transmission rate (MVTR) in some applications. For example, the MVTR may be at least 250 grams per square meter per twenty-four hours in some embodiments, measured using an upright cup technique according to ASTM E96/E96M Upright Cup Method at 38°C and 10% relative humidity (RH). In some embodiments, an MVTR up to 5,000 grams per square meter per twenty-four hours may provide effective breathability and mechanical properties.

In some example embodiments, the cover 125 may be a non-porous polymer drape or film, such as a polyurethane film, that is permeable to water vapor but impermeable to liquid. Such drapes typically have a thickness in the range of 25-50 microns. For permeable materials, the permeability generally should be low enough that a desired negative pressure may be maintained. The cover 125 may comprise, for example, one or more of the following materials: polyurethane (PU), such as hydrophilic polyurethane; cellulosics; hydrophilic polyamides; polyvinyl alcohol; polyvinyl pyrrolidone; hydrophilic acrylics; silicones, such as hydrophilic silicone elastomers; natural rubbers; polyisoprene; styrene butadiene rubber; chloroprene rubber; polybutadiene; nitrile rubber; butyl rubber; ethylene propylene rubber; ethylene propylene diene monomer; chlorosulfonated polyethylene; polysulfide rubber; ethylene vinyl acetate (EVA); co-polyester; and polyether block polymide copolymers. Such materials are commercially available as, for example, Tegaderm^{®} drape, commercially available from 3M Company, Minneapolis Minnesota; polyurethane (PU) drape, commercially available from Avery Dennison Corporation, Pasadena, California; polyether block polyamide copolymer (PEBAX), for example, from Arkema S.A., Colombes, France; and Inspire 2301 and Inpsire 2327 polyurethane films, commercially available from Expopack Advanced Coatings, Wrexham, United Kingdom. In some embodiments, the cover 125 may comprise INSPIRE 2301 having an MVTR (upright cup technique) of 2600 g/m²/24 hours and a thickness of about 30 microns.

An attachment device may be used to attach the cover 125 to an attachment surface, such as undamaged epidermis, a gasket, or another cover. The attachment device may take many forms. For example, an attachment device may be a medically-acceptable, pressure-sensitive adhesive configured to bond the cover 125 to epidermis around a tissue site. In some embodiments, for example, some or all of the cover 125 may be coated with an adhesive, such as an acrylic adhesive, which may have a coating weight of about 25-65 grams per square meter (g.s.m.). Thicker adhesives, or combinations of adhesives, may be applied in some embodiments to improve the seal and reduce leaks. Other example embodiments of an attachment device may include a double-sided tape, paste, hydrocolloid, hydrogel, silicone gel, or organogel.

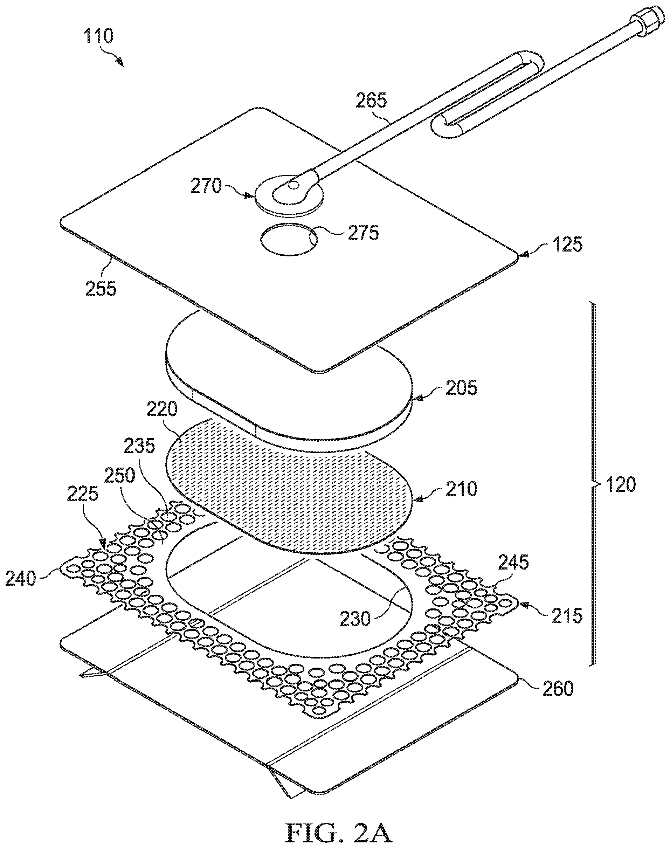

Figure 2A is an assembly view of an example of the dressing 110 of Figure 1, illustrating additional details that may be associated with some embodiments in which the tissue interface 120 comprises more than one layer. In the example of Figure 2A, the tissue interface 120 comprises a first layer 205, a second layer 210, and an optional third layer 215. In some embodiments, the first layer 205 may be disposed adjacent to the second layer 210, and the third layer 215 may also be disposed adjacent to the second layer 210 opposite the first layer 205. For example, the first layer 205 and the second layer 210 may be stacked so that the first layer 205 is in contact with the second layer 210. The first layer 205 may also be bonded to the second layer 210 in some embodiments. In some embodiments, the second layer 210 may be coextensive with a face of the first layer 205. In some embodiments, at least some portion of the third layer 215 may be bonded to the second layer 210.

The first layer 205 generally comprises or consists essentially of a manifold or a manifold layer, which provides a means for collecting or distributing fluid across the tissue interface 120 under pressure and/or under reduced pressure. For example, the first layer 205 may be adapted to receive negative pressure from a source and distribute negative pressure through multiple apertures across the tissue interface 120, which may have the effect of collecting fluid from across a tissue site and drawing the fluid toward the source. In some embodiments, the fluid path may be reversed, or a secondary fluid path may be provided to facilitate delivering fluid, such as from a source of instillation solution, across the tissue interface 120.

In some illustrative embodiments, the pathways of the first layer 205 may be interconnected to improve distribution or collection of fluids. In some illustrative embodiments, the first layer 205 may comprise or consist essentially of a porous material having interconnected fluid pathways. Examples of suitable porous material that comprise or can be adapted to form interconnected fluid pathways (e.g., channels) may include cellular foam, including open-cell foam such as reticulated foam; porous tissue collections; and other porous material such as gauze or felted mat that generally include pores, edges, and/or walls. Liquids, gels, and other foams may also include or be cured to include apertures and fluid pathways. In some embodiments, the first layer 205 may additionally or alternatively comprise projections that form interconnected fluid pathways. For example, the first layer 205 may be molded to provide surface projections that define interconnected fluid pathways.

In some embodiments, the first layer 205 may comprise or consist essentially of a reticulated foam having pore sizes and free volume that may vary according to needs of a prescribed therapy. For example, a reticulated foam having a free volume of at least 90% may be suitable for many therapy applications, and a foam having an average pore size in a range of 400-600 microns may be particularly suitable for some types of therapy. The tensile strength of the first layer 205 may also vary according to needs of a prescribed therapy. For example, the tensile strength of a foam may be increased for instillation of topical treatment solutions. The 25% compression load deflection of the first layer 205 may be at least 0.35 pounds per square inch, and the 65% compression load deflection may be at least 0.43 pounds per square inch. In some embodiments, the tensile strength of the first layer 205 may be at least 10 pounds per square inch. The first layer 205 may have a tear strength of at least 2.5 pounds per inch. In some embodiments, the first layer 205 may be a foam comprised of polyols such as polyester or polyether, isocyanate such as toluene diisocyanate, and polymerization modifiers such as amines and tin compounds. In some examples, the first layer 205 may be a reticulated polyurethane foam such as used in GRANUFOAM^{™} dressing or V.A.C. VERAFLO^{™} dressing, both available from KCI of San Antonio, Texas.

Other suitable materials for the first layer 205 may include non-woven fabrics (Libeltex, Freudenberg), three-dimensional (3D) polymeric structures (molded polymers, embossed and formed films, and fusion bonded films [Supracore]), and mesh, for example.

In some examples, the first layer 205 may include a 3D textile, such as various textiles commercially available from Baltex, Muller, and Heathcoates. A 3D textile of polyester fibers may be particularly advantageous for some embodiments. For example, the first layer 205 may comprise or consist essentially of a three-dimensional weave of polyester fibers. In some embodiments, the fibers may be elastic in at least two dimensions. A puncture-resistant fabric of polyester and cotton fibers having a weight of about 650 grams per square meter and a thickness of about 1-2 millimeters may be particularly advantageous for some embodiments. Such a puncture-resistant fabric may have a warp tensile strength of about 330-340 kilograms and a weft tensile strength of about 270-280 kilograms in some embodiments. Another particularly suitable material may be a polyester spacer fabric having a weight of about 470 grams per square meter, which may have a thickness of about 4-5 millimeters in some embodiments. Such a spacer fabric may have a compression strength of about 20-25 kilopascals (at 40% compression). Additionally or alternatively, the first layer 205 may comprise or consist of a material having substantial linear stretch properties, such as a polyester spacer fabric having 2-way stretch and a weight of about 380 grams per square meter. A suitable spacer fabric may have a thickness of about 3-4 millimeters and may have a warp and weft tensile strength of about 30-40 kilograms in some embodiments. The fabric may have a close-woven layer of polyester on one or more opposing faces in some examples. In some embodiments, a woven layer may be advantageously disposed on a first layer 205 to face a tissue site.

The first layer 205 generally has a first planar surface and a second planar surface opposite the first planar surface. The thickness of the first layer 205 between the first planar surface and the second planar surface may also vary according to needs of a prescribed therapy. For example, the thickness of the first layer 205 may be decreased to relieve stress on other layers and to reduce tension on peripheral tissue. The thickness of the first layer 205 can also affect the conformability of the first layer 205. In some embodiments, a suitable foam may have a thickness in a range of about 5 millimeters to 10 millimeters. Fabrics, including suitable 3D textiles and spacer fabrics, may have a thickness in a range of about 2 millimeters to about 8 millimeters.

The second layer 210 may comprise or consist essentially of a means for controlling or managing fluid flow. In some embodiments, the second layer 210 may be a fluid control layer comprising or consisting essentially of a liquid-impermeable, elastomeric material. For example, the second layer 210 may comprise or consist essentially of a polymer film, such as a polyurethane film, a polyethylene film, an acrylic film or a polyester film. In some embodiments, the second layer 210 may comprise or consist essentially of the same material as the cover 125. The second layer 210 may also have a smooth or matte surface texture in some embodiments. A glossy or shiny finish finer or equal to a grade B3 according to the SPI (Society of the Plastics Industry) standards may be particularly advantageous for some applications. In some embodiments, variations in surface height may be limited to acceptable tolerances. For example, the surface of the second layer 210 may have a substantially flat surface, with height variations limited to 0.2 millimeters over a centimeter.

In some embodiments, the second layer 210 may be hydrophobic. The hydrophobicity of the second layer 210 may vary but may have a contact angle with water of at least ninety degrees in some embodiments. In some embodiments the second layer 210 may have a contact angle with water of no more than 150 degrees. For example, in some embodiments, the contact angle of the second layer 210 may be in a range of at least 90 degrees to about 120 degrees, or in a range of at least 120 degrees to 150 degrees. Water contact angles can be measured using any standard apparatus. Although manual goniometers can be used to visually approximate contact angles, contact angle measuring instruments can often include an integrated system involving a level stage, liquid dropper such as a syringe, camera, and software designed to calculate contact angles more accurately and precisely, among other things. Non-limiting examples of such integrated systems may include the FTÅ125, FTÅ200, FTÅ2000, and FTÅ4000 systems, all commercially available from First Ten Angstroms, Inc., of Portsmouth, VA, and the DTA25, DTA30, and DTA100 systems, all commercially available from Kruss GmbH of Hamburg, Germany. Unless otherwise specified, water contact angles herein are measured using deionized and distilled water on a level sample surface for a sessile drop added from a height of no more than 5 cm in air at 20-25°C and 20-50% relative humidity. Contact angles herein represent averages of 5-9 measured values, discarding both the highest and lowest measured values. The hydrophobicity of the second layer 210 may be further enhanced with a hydrophobic coating of other materials, such as silicones and fluorocarbons, either as coated from a liquid, or plasma coated.

The second layer 210 may also be suitable for welding to other layers, including the first layer 205. For example, the second layer 210 may be adapted for welding to polyurethane foams using heat, radio frequency (RF) welding, or other methods to generate heat such as ultrasonic welding. RF welding may be particularly suitable for more polar materials, such as polyurethane, polyamides, polyesters and acrylates. Sacrificial polar interfaces may be used to facilitate RF welding of less polar film materials, such as polyethylene.

The area density of the second layer 210 may vary according to a prescribed therapy or application. In some embodiments, an area density of less than 40 grams per square meter may be suitable, and an area density of about 20-30 grams per square meter may be particularly advantageous for some applications.

In some embodiments, for example, the second layer 210 may comprise or consist essentially of a hydrophobic polymer, such as a polyethylene film. The simple and inert structure of polyethylene can provide a surface that interacts little, if any, with biological tissues and fluids, providing a surface that may encourage the free flow of liquids and low adherence, which can be particularly advantageous for many applications. Other suitable polymeric films include polyurethanes, acrylics, polyolefin (such as cyclic olefin copolymers), polyacetates, polyamides, polyesters, copolyesters, PEBAX block copolymers, thermoplastic elastomers, thermoplastic vulcanizates, polyethers, polyvinyl alcohols, polypropylene, polymethylpentene, polycarbonate, styrenics, silicones, fluoropolymers, and acetates. A thickness between 20 microns and 100 microns may be suitable for many applications. Films may be clear, colored, or printed. More polar films suitable for laminating to a polyethylene film include polyamide, co-polyesters, ionomers, and acrylics. To aid in the bond between a polyethylene and polar film, tie layers may be used, such as ethylene vinyl acetate, or modified polyurethanes. An ethyl methyl acrylate (EMA) film may also have suitable hydrophobic and welding properties for some configurations.

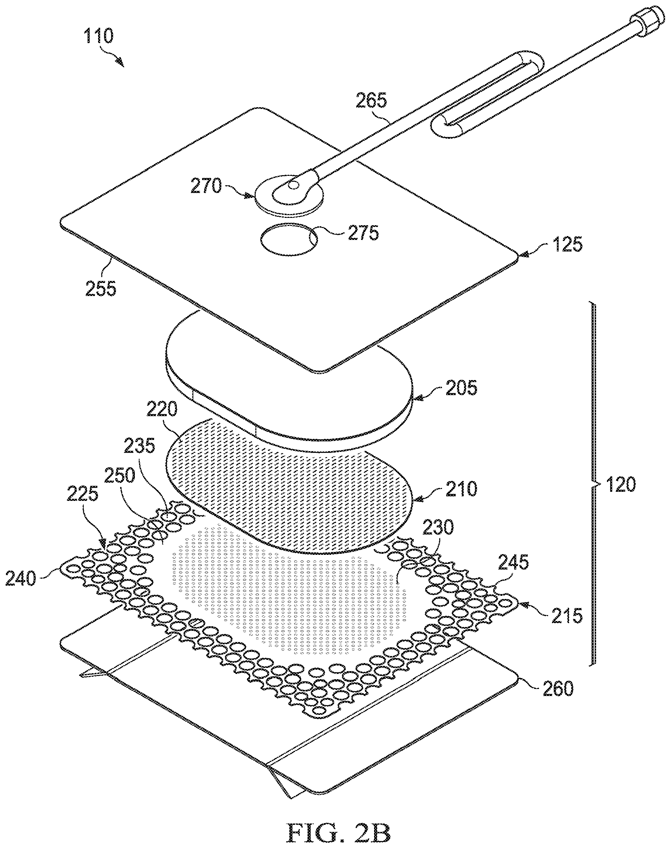

As illustrated in the example of Figure 2A and 2B, the second layer 210 may have one or more fluid restrictions 220, which can be distributed uniformly or randomly across the second layer 210. The fluid restrictions 220 may be bi-directional and pressure-responsive and reversibly openable. For example, each of the fluid restrictions 220 generally may comprise or consist essentially of an elastic passage that is normally unstrained to substantially reduce liquid flow, and can expand or open in response to a pressure gradient. In some embodiments, the fluid restrictions 220 may comprise or consist essentially of perforations in the second layer 210. Perforations may be formed by removing material from the second layer 210. For example, perforations may be formed by cutting through the second layer 210, which may also deform the edges of the perforations in some embodiments. In the absence of a pressure gradient across the perforations, the passages may be sufficiently small to form a seal or fluid restriction, which can substantially reduce or prevent liquid flow. Additionally or alternatively, one or more of the fluid restrictions 220 may be an elastomeric valve that is normally closed when unstrained to substantially prevent liquid flow, and can open in response to a pressure gradient. A fenestration in the second layer 210 may be a suitable valve for some applications. Fenestrations may also be formed by removing material from the second layer 210, but the amount of material removed, and the resulting dimensions of the fenestrations may be up to an order of magnitude less than perforations, and may not deform the edges.

For example, some embodiments of the fluid restrictions 220 may comprise or consist essentially of one or more slits, slots or combinations of slits and slots in the second layer 210. In some examples, the fluid restrictions 220 may comprise or consist of linear slots having a length less than 4 millimeters and a width less than 1 millimeter. The length may be at least 2 millimeters, and the width may be at least 0.4 millimeters in some embodiments. A length of about 3 millimeters and a width of about 0.8 millimeters may be particularly suitable for many applications, and a tolerance of about 0.1 millimeter may also be acceptable. Such dimensions and tolerances may be achieved with a laser cutter, for example. Slots of such configurations may function as imperfect valves that substantially reduce liquid flow in a normally closed or resting state. For example, such slots may form a flow restriction without being completely closed or sealed. The slots can expand or open wider in response to a pressure gradient to allow increased liquid flow.

In some embodiments, citric acid may be present on and/or applied to one or more surfaces of the second layer 210 to form a coating. For example, the second layer 210 may be a polyurethane or polyethylene film having a citric acid coating on one or more surfaces of the film. The second layer 210 may be partially coated or entirely coated with citric acid.

A variety of known methods exist to coat or apply the second layer 210 with citric acid, such as pattern coating and deposition. In some embodiments, citric acid is covalently bound to one or more surfaces of the second layer 210. For example, surface functionalization technology can be used to covalently bind citric acid to one or more surfaces of the second layer 210, such as the OptoDex@ system commercially available from CSEM Landquart, Landquart, Switzerland. Alternatively, a UV cure can be used to covalently bind the citric acid coating to one or more surfaces of the second layer 210.

Additionally or alternatively, citric acid may be non-covalently bound to one or more surfaces of the second layer 210. For example, a citric acid containing solution may be applied and then dried on one or more surfaces of the second layer 210.

Additionally or alternatively, the second layer 210 may comprise citric acid at least partially encapsulated in a polymer, the encapsulated citric acid being coated on the surface of the second layer 210. In some instances, the second layer 210 may comprise citric acid at least partially encapsulated in collagen and oxidized regenerated cellulose, the encapsulated citric acid being coated on a surface of the second layer 210.

Additionally or alternatively, the second layer 210 may comprise citric acid. In some embodiments, citric acid may be added to the preparation (e.g. a master batch) when making the second layer 210 to incorporate citric acid into the second layer 210. In some embodiments, the second layer 210 comprises about 0.5% to about 15% citric acid, preferably about 1% to about 10% citric acid. As another example, the second layer 210 may contain citric acid incorporated into a polymer component of the layer. The polymer may be a water-soluble or water-sensitive polymer. As used herein "water-soluble" refers to any material having a solubility in water of 10 mg/L and greater at standard temperature and pressure. As used herein "water-sensitive" refers to a material that undergoes a physical or chemical change when contacted with water. In some instances, the citric acid is homogenously contained within the second layer 210 and/or homogenously distributed on the surface of the second layer 210.

In some embodiments, the second layer 210 comprises citric acid and citric acid is present on and/or coated on one or more surfaces of the second layer 210.

The optional third layer 215 may comprise or consist essentially of a sealing layer formed from a soft, pliable material suitable for providing a fluid seal with a tissue site, such as a suitable gel material, and may have a substantially flat surface. For example, the third layer 215 may comprise, without limitation, a soft polymer gel such as silicone gel, hydrogel, polyurethane gel, polyolefin gel, hydrogenated styrenic copolymer gel, a foamed gel; and/or a soft closed cell foam such as a polyurethane, a polyolefin or a hydrogenated styrenic copolymer; and/or a hydrocolloid dressing containing gel-forming agents. The third layer 215 may be partially or entirely coated with an adhesive. In some embodiments, the third layer 215 may have a thickness between about 200 microns (µm) and about 1000 microns (µm). In some embodiments, the third layer 215 may have a hardness between about 5 Shore OO and about 80 Shore OO. Further, the third layer 215 may be comprised of hydrophobic or hydrophilic materials.

In some embodiments, the third layer 215 may be a hydrophobic-coated material. For example, the third layer 215 may be formed by coating a spaced material, such as, for example, a woven, a non-woven, a molded, or an extruded mesh with a hydrophobic material. The hydrophobic material for the coating may be a soft silicone, for example.

In some embodiments, citric acid may be present on and/or coated on one or more surfaces of the third layer 215. For example, the third layer 215 may be a silicone layer having citric acid coated on one or more surfaces of the silicone layer. The third layer 215 may be partially coated or entirely coated with citric acid.

A variety of known methods exist to coat or apply the third layer 215 with citric acid, such as pattern coating and deposition. In some embodiments, citric acid is covalently bound to one or more surfaces of the third layer 215. For example, surface functionalization technology can be used to covalently bind citric acid to one or more surfaces of the third layer 215, such as the OptoDex@ system commercially available from CSEM Landquart, Landquart, Switzerland.

Additionally or alternatively, citric acid may be non-covalently bound to one or more surfaces of the third layer 215. For example, citric acid may be applied and then dried on one or more surfaces of the third layer 215.

Additionally or alternatively, the third layer 215 may comprise citric acid at least partially encapsulated in a polymer, the encapsulated citric acid being coated on the surface of the third layer 215. In some instances, the third layer 215 may comprise citric acid at least partially encapsulated in collagen and oxidized regenerated cellulose, the encapsulated citric acid being coated on a surface of the third layer 215.

Additionally or alternatively, the third layer 215 may comprise citric acid. In some embodiments, citric acid may be added to the preparation (e.g. a master batch) when making the third layer 215 to incorporate citric acid into the third layer 215. In some embodiments, the third layer 215 comprises about 0.5% to about 15% citric acid, preferably about 1% to about 10% citric acid. As another example, the third layer 215 may contain citric acid incorporated into a polymer component of the layer. The polymer may be a water-soluble or water-sensitive polymer as defined above. In some instances, the citric acid is homogenously contained within the third layer 215 and/or homogenously distributed on the surface of the third layer 215.

In some embodiments, the third layer 215 comprises citric acid and citric acid is present on and/or coated on one or more surfaces of the third layer 215.

In some embodiments, both the second layer 210 and the third layer 215 have citric acid incorporated therein and/or have one or two surfaces that have a citric acid coating present thereon, or any combination of coating and incorporation.

The third layer 215 may have a periphery 225 surrounding or around a treatment area, which may have one or more treatment apertures. In the example of Figure 2A, the third layer 215 has a treatment aperture 230, and apertures 235 in the periphery 225 disposed around the treatment aperture 230. The treatment aperture 230 may be complementary or correspond to a surface area of the first layer 205 in some examples. For example, the treatment aperture 230 may form a frame, window, or other opening around a surface of the first layer 205. The third layer 215 may also have corners 240 and edges 245. The corners 240 and the edges 245 may be part of the periphery 225. The third layer 215 may have an interior border 250 around the treatment aperture 230, which can at least partially define a treatment area. The interior border 250 may be substantially free of the apertures 235, as illustrated in the example of Figure 2A. In some examples, as illustrated in Figure 2A, the treatment aperture 230 may be symmetrical and centrally disposed in the third layer 215, forming an open central window. In other examples, the treatment area may comprise or be defined by a plurality of treatment apertures, which may be substantially smaller than the treatment aperture 230 as illustrated in Figure 2B. In further embodiments, only the treatment area of the third layer 215 may comprise citric acid and/or have citric acid present on the surface of the treatment area, but not in or on the remaining part of the third layer 215. For example, in Figure 2A the interior border 250 may comprise citric acid and/or have citric acid present on the surface of the interior border 250.

In some embodiments, the third layer 215 is called a perforated layer. The apertures 235 may be formed by cutting, perforating, or by application of local RF or ultrasonic energy, for example, or by other suitable techniques for forming an opening or perforation in the third layer 215. The apertures 235 may have a uniform distribution pattern or may be randomly distributed on the third layer 215. The apertures 235 in the third layer 215 may have many shapes, including circles, squares, stars, ovals, polygons, slits, complex curves, rectilinear shapes, triangles, for example, or may have some combination of such shapes.

Each of the apertures 235 may have uniform or similar geometric properties. For example, in some embodiments, each of the apertures 235 may be circular apertures, having substantially the same diameter. In some embodiments, each of the apertures 235 may have a diameter of about 1 millimeter to about 50 millimeters. In other embodiments, the diameter of each of the apertures 235 may be about 1 millimeter to about 20 millimeters.

In other embodiments, geometric properties of the apertures 235 may vary. For example, the diameter of the apertures 235 may vary depending on the position of the apertures 235 in the third layer 215. For example, in some embodiments, the apertures 235 disposed in the periphery 225 may have a diameter between about 5 millimeters and about 10 millimeters. A range of about 7 millimeters to about 9 millimeters may be suitable for some examples. In some embodiments, the apertures 235 disposed in the corners 240 may have a diameter between about 7 millimeters and about 8 millimeters.

At least one of the apertures 235 in the periphery 225 of the third layer 215 may be positioned at the edges 245 of the periphery 225 and may have an interior cut open or exposed at the edges 245 that is in fluid communication in a lateral direction with the edges 245. The lateral direction may refer to a direction toward the edges 245 and in the same plane as the third layer 215. As shown in the example of Figure 2A, the apertures 235 in the periphery 225 may be positioned proximate to or at the edges 245 and in fluid communication in a lateral direction with the edges 245. The apertures 235 positioned proximate to or at the edges 245 may be spaced substantially equidistant around the periphery 225 as shown in the example of Figure 2A. Alternatively, the spacing of the apertures 235 proximate to or at the edges 245 may be irregular.

As illustrated in the example of Figure 2A, the dressing 110 may further include an attachment device, such as an adhesive 255. The adhesive 255 may be, for example, a medically-acceptable, pressure-sensitive adhesive that extends about a periphery, a portion, or an entire surface of the cover 125. In some embodiments, for example, the adhesive 255 may be an acrylic adhesive having a coating weight between 25-65 grams per square meter (g.s.m.). Thicker adhesives, or combinations of adhesives, may be applied in some embodiments to improve the seal and reduce leaks. In some embodiments, such a layer of the adhesive 255 may be continuous or discontinuous. Discontinuities in the adhesive 255 may be provided by apertures or holes (not shown) in the adhesive 255. The apertures or holes in the adhesive 255 may be formed after application of the adhesive 255 or by coating the adhesive 255 in patterns on a carrier layer, such as, for example, a side of the cover 125. Apertures or holes in the adhesive 255 may also be sized to enhance the MVTR of the dressing 110 in some example embodiments.

As illustrated in the example of Figure 2A, in some embodiments, the dressing 110 may include a release liner 260 to protect the adhesive 255 prior to use. The release liner 260 may also provide stiffness to assist with, for example, deployment of the dressing 110. The release liner 260 may be, for example, a casting paper, a film, or a polymer such as polyethylene. Further, in some embodiments, the release liner 260 may be a polyester material such as polyethylene terephthalate (PET), or similar polar semi-crystalline polymer. The use of a polar semi-crystalline polymer for the release liner 260 may substantially preclude wrinkling or other deformation of the dressing 110. For example, the polar semi-crystalline polymer may be highly orientated and resistant to softening, swelling, or other deformation that may occur when brought into contact with components of the dressing 110, or when subjected to temperature or environmental variations, or sterilization. Further, a release agent may be disposed on a side of the release liner 260 that is configured to contact the second layer 210. For example, the release agent may be a silicone coating and may have a release factor suitable to facilitate removal of the release liner 260 by hand and without damaging or deforming the dressing 110. In some embodiments, the release agent may be a fluorocarbon or a fluorosilicone, for example. In other embodiments, the release liner 260 may be uncoated or otherwise used without a release agent.

Figure 2A also illustrates one example of a fluid conductor 265 and a dressing interface 270. As shown in the example of Figure 2A, the fluid conductor 265 may be a flexible tube, which can be fluidly coupled on one end to the dressing interface 270. The dressing interface 270 may be an elbow connector, as shown in the example of Figure 2A, which can be placed over an aperture 275 in the cover 125 to provide a fluid path between the fluid conductor 265 and the tissue interface 120.

One or more of the components of the dressing 110 may additionally be treated with an antimicrobial agent in some embodiments. For example, the first layer 205 may be a foam, mesh, or non-woven coated with an antimicrobial agent. In some embodiments, the first layer may comprise antimicrobial elements, such as fibers coated with an antimicrobial agent. Additionally or alternatively, some embodiments of the second layer 210 may be a polymer coated or mixed with an antimicrobial agent. In other examples, the fluid conductor 265 may additionally or alternatively be treated with one or more antimicrobial agents. Suitable antimicrobial agents may include, for example, metallic silver, PHMB, iodine or its complexes and mixes such as povidone iodine, copper metal compounds, chlorhexidine, or some combination of these materials.

Additionally, the dressing 110 in the example of Figure 2A may also comprise a first optional fluid wicking layer (not shown) between the cover 125 and the first layer 205; and/or a second optional fluid wicking layer (now shown) between the first layer 205 and the second layer 210. The optional fluid wicking layers may passively encourage the distribution of fluid evenly across the dressing 110 and increase absorption into the first layer 205. The first and the second optional fluid wicking layer may comprise a hydrophilic non-woven and/or a hydrophilic woven or mesh. In some embodiments, the first and/or the second optional fluid wicking layer may have a citric acid coating present on one or more surfaces thereof as described herein.

Additionally, the dressing 110 may also comprise an additional absorbent layer and/or a wicking layer comprising an absorbent.

Additionally, the dressing 110 may also comprise collagen and oxidized regenerated cellulose as a distinct layer in the dressing 110 or present on one or more surfaces of the second layer 210 and/or third layer 215. In some instances, the collagen and oxidized regenerated cellulose may at least partially encapsulate citric acid.

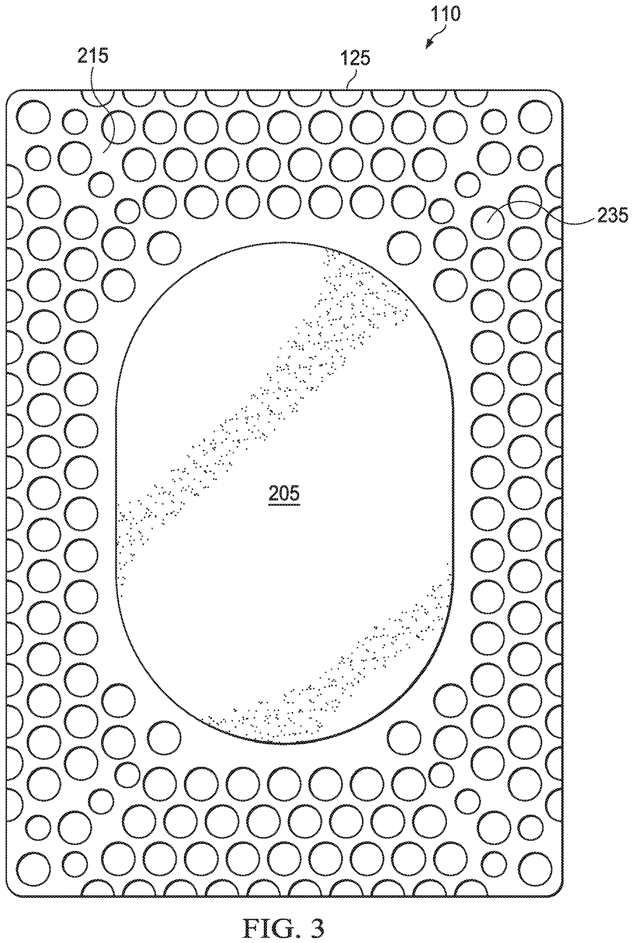

Figure 3 is a top view of the dressing 110 in the example of Figure 2A, as assembled, illustrating additional details that may be associated with some embodiments. As illustrated in the example of Figure 2A, the cover 125 and the third layer 215 may have substantially the same perimeter shape and dimensions, so that the cover 125 and the third layer 215 are coextensive in some examples. The cover 125 may be substantially transparent, allowing visibility of the apertures 235 in some embodiments. The first layer 205 may be centrally disposed over the third layer 215, such as over the treatment aperture 230 (not visible in Figure 3). The cover 125 may be disposed over the first layer 205 and coupled to the third layer 215 around the first layer 205 so that at least some of the adhesive 255 can be disposed adjacent to the apertures 235.

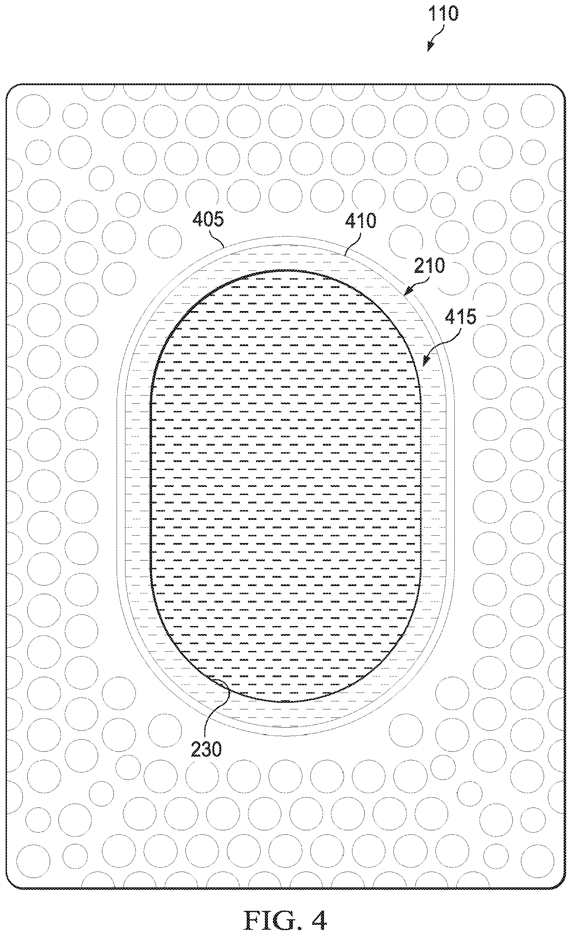

Figure 4 is a bottom view of the dressing 110 in the example of Figure 2A, as assembled, illustrating additional details that may be associated with some embodiments. As illustrated in the example of Figure 4, a substantial number of the fluid restrictions 220 may be aligned or otherwise exposed through the treatment aperture 230, and at least some portion of the first layer 205 may be disposed adjacent to the fluid restrictions 220 opposite the treatment aperture 230. In some embodiments, the first layer 205 and the second layer 210 may be substantially aligned with the treatment aperture 230 or may extend across the treatment aperture 230.

Additionally, the first layer 205 may have a first edge 405, and the second layer 210 may have a second edge 410. In some examples, the first edge 405 and the second edge 410 may have substantially the same shape so that adjacent faces of the first layer 205 and the second layer 210 are geometrically similar. The first edge 405 and the second edge 410 may also be congruent in some examples, so that adjacent faces of the first layer 205 and the second layer 210 are substantially coextensive and have substantially the same surface area. In the example of Figure 4, the first edge 405 defines a larger face of the first layer 205 than the face of the second layer 210 defined by the second edge 410, and the larger face of the first layer 205 extends past the smaller face of the second edge 410.

The faces defined by the first edge 405, the second edge 410, or both may also be geometrically similar to the treatment aperture 230 in some embodiments, as illustrated in the example of Figure 4, and may be larger than the treatment aperture 230. The third layer 215 may have an overlay margin 415 around the treatment aperture 230, which may have an additional adhesive disposed therein. As illustrated in the example of Figure 4, the treatment aperture 230 may be an ellipse or a stadium in some embodiments. The treatment aperture 230 may have an area that is equal to about 20% to about 80% of the area of the third layer 215 in some examples. The treatment aperture 230 may also have an area that is equal to about 20% to about 100% of the area of a face defined by the first edge 405 of the first layer 205. A width of about 90 millimeters to about 110 millimeters and a length of about 150 millimeters to about 160 millimeters may be suitable for some embodiments of the treatment aperture 230. For example, the width of the treatment aperture 230 may be about 100 millimeters, and the length may be about 155 millimeters. In some embodiments, a suitable width for the overlay margin 415 may be about 2 millimeters to about 3 millimeters. For example, the overlay margin 415 may be coextensive with an area defined between the treatment aperture 230 and the first edge 405, and the adhesive may secure the first layer 205, the second layer 210, or both to the third layer 215.

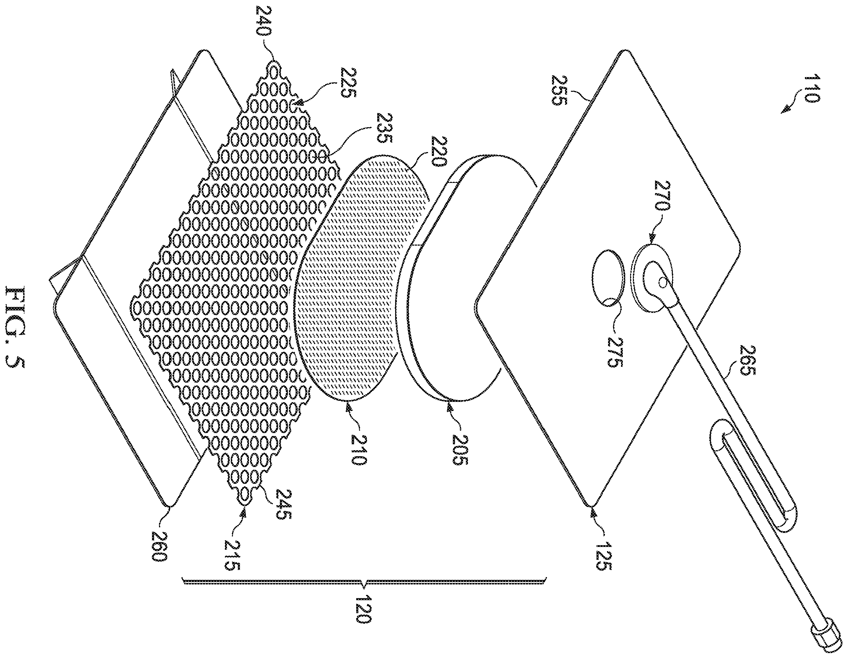

Figure 5 is an assembly view of another example of the dressing 110 of Figure 1, illustrating additional details that may be associated with some embodiments. As illustrated in Figure 5, the apertures 235 may be distributed in a uniform pattern across the third layer 215 in some examples. At least some of the apertures 235 may define or provide a treatment area, and at least some of the apertures 235 may be treatment apertures.

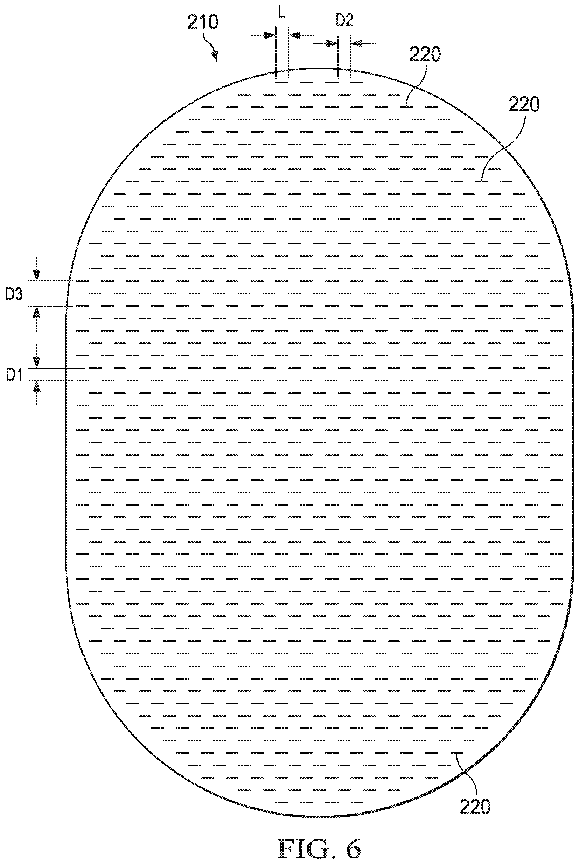

Figure 6 is a schematic view of an example of the second layer 210, illustrating additional details that may be associated with some embodiments. As illustrated in the example of Figure 6, the fluid restrictions 220 may each consist essentially of one or more slits having a length L. A length of about 3 millimeters may be particularly suitable for some embodiments. Figure 6 additionally illustrates an example of a uniform distribution pattern of the fluid restrictions 220. In Figure 6, the fluid restrictions 220 are substantially coextensive with the second layer 210 and are distributed across the second layer 210 in a grid of parallel rows and columns, in which the slits are also mutually parallel to each other. In some embodiments, the rows may be spaced a distance D1. A distance of about 3 millimeters on center may be suitable for some embodiments. The fluid restrictions 220 within each of the rows may be spaced a distance D2, which may be about 3 millimeters on center in some examples. The fluid restrictions 220 in adjacent rows may be aligned or offset in some embodiments. For example, adjacent rows may be offset, as illustrated in Figure 6, so that the fluid restrictions 220 are aligned in alternating rows and separated by a distance D3, which may be about 6 millimeters in some embodiments. The spacing of the fluid restrictions 220 may vary in some embodiments to increase the density of the fluid restrictions 220 according to therapeutic requirements.

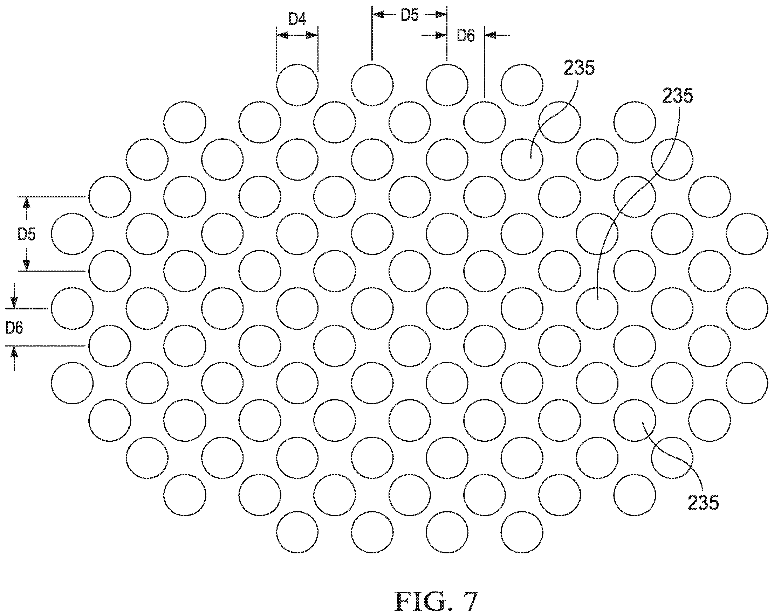

Figure 7 is a schematic view of an example configuration of the apertures 235, illustrating additional details that may be associated with some embodiments of the third layer 215. In the example of Figure 7, the apertures 235 are generally circular and have a diameter D4, which may be about 6 millimeters to about 8 millimeters in some embodiments. A diameter D4 of about 7 millimeters may be particularly suitable for some embodiments. Figure 7 also illustrates an example of a uniform distribution pattern of the apertures 235. In Figure 7, the apertures 235 are distributed across the third layer 215 in a grid of parallel rows and columns. Within each row and column, the apertures 235 may be equidistant from each other, as illustrated in the example of Figure 7. Figure 7 illustrates one example configuration that may be particularly suitable for many applications, in which the apertures 235 are spaced a distance D5 apart along each row and column, with an offset of D6. In some examples, the distance D5 may be about 9 millimeters to about 10 millimeters, and the offset D6 may be about 8 millimeters to about 9 millimeters.

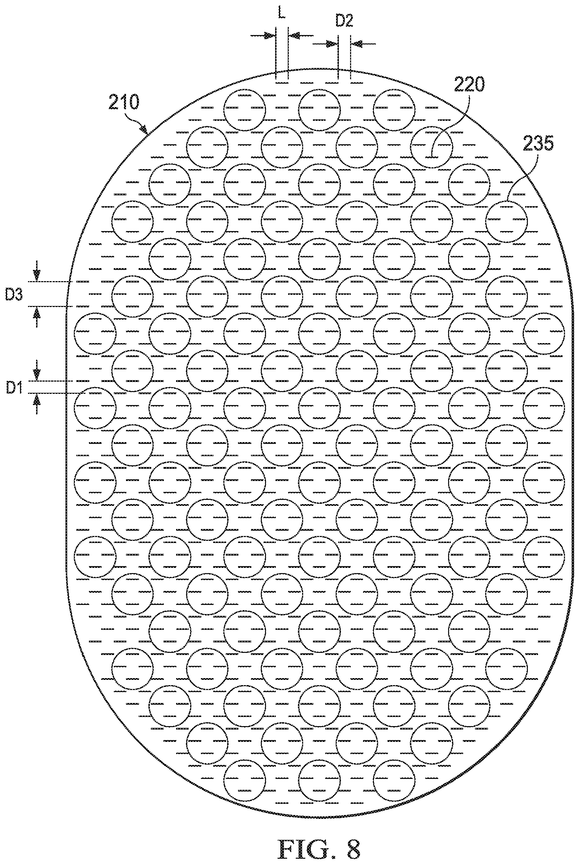

Figure 8 is a schematic view of the apertures 235 in the example of Figure 7 overlaid on the second layer 210 of Figure 6, illustrating additional details that may be associated with some example embodiments of the tissue interface 120. For example, as illustrated in Figure 8, more than one of the fluid restrictions 220 may be aligned, overlapping, in registration with, or otherwise fluidly coupled to the apertures 235 in some embodiments. In some embodiments, one or more of the fluid restrictions 220 may be only partially registered with the apertures 235. The apertures 235 in the example of Figure 8 are generally sized and configured so that at least four of the fluid restrictions 220 are registered with each one of the apertures 235. In other examples, one or more of the fluid restrictions 220 may be registered with more than one of the apertures 235. For example, any one or more of the fluid restrictions 220 may be a perforation or a fenestration that extends across two or more of the apertures 235. Additionally or alternatively, one or more of the fluid restrictions 220 may not be registered with any of the apertures 235.

As illustrated in the example of Figure 8, the apertures 235 may be sized to expose a portion of the second layer 210, the fluid restrictions 220, or both through the third layer 215. The apertures 235 in the example of Figure 8 are generally sized to expose more than one of the fluid restrictions 220. Some or all of the apertures 235 may be sized to expose two or three of the fluid restrictions 220. In some examples, the length of each of the fluid restrictions 220 may be substantially smaller than the diameter of each of the apertures 235. More generally, the average dimensions of the fluid restrictions 220 are substantially smaller than the average dimensions of the apertures 235. In some examples, the apertures 235 may be elliptical, and the length of each of the fluid restrictions 220 may be substantially smaller than the major axis or the minor axis. In some embodiments, though, the dimensions of the fluid restrictions 220 may exceed the dimensions of the apertures 235, and the size of the apertures 235 may limit the effective size of the fluid restrictions 220 exposed to the lower surface of the dressing 110.

### Example Methods to Make

In additional embodiments, methods of making the dressing 110 are also disclosed herein.

### Incorporating Citric Acid

In some embodiments, citric acid is incorporated into the second layer 210 (e.g. the fluid control layer) and/or the third layer 215 (e.g. the sealing layer) by adding citric acid to a polymer solution used to form the second layer 210 and/or the third layer 215 prior to casting the second layer 210 and/or the third layer 215. For example, citric acid may be introduced into a master batch used to prepare the second layer 210 and/or the third layer 215.

In some embodiments, about 0.5% w/w to about 15% w/w, preferably about 1% w/w to about 4% w/w of citric acid can be added to a polymer solution used to form the second layer 210. The polymer solution used to form the second layer 210 may comprise polyvinyl alcohol, polyvinyl pyrrolidone, polyethylene oxide, a soluble salt of carboxymethyl cellulose, polyacrylic acid, polymethyl acrylic acid and a combination thereof. The polymer solution containing citric acid may then be coated or cast onto a release liner and dried to from the second layer 210.

For example, in some embodiments, the second layer 210 containing citric acid may be formed from a solvent cast process where the polymer used to form the second layer 210 is dissolved into a solvent to form a polymer solution and the polymer solution can be applied to a roll where evaporation occurs, forming a complete polymer film. During this process, citric acid can be added to the solvent. Examples include polyvinyl acetate dissolved in alcohols or ketones (and their mixtures) along with citric acid. Other polymers include acrylics and polyurethanes also dissolved in ketones, ethers, acetates and furans. Additionally, the second layer 210 may also be formed from casting an aqueous polymer emulsion or dispersion (such as a polyurethane dispersion (PUD)), where the citric acid may be dissolved in the aqueous phase and will co-cast with the polymer during the film forming process.

Additionally or alternatively, about 0.5% w/w to about 15% w/w, preferably about 1% w/w to about 4% w/w, of citric acid can be added to a polymer solution used to form the third layer 215. The polymer solution used to form the third layer 215 may comprise silicone. A range of silicones may be suitable to form the second layer 210. For example, the polymer solution containing citric acid may be cured into a soft gel by using a two-part crosslinking system, such as a platinum catalyzed crosslinking system. For example, part 1 may contain a vinyl functionality and a platinum catalyst, and part 2 may contain the polymer solution having a tertiary hydrogen functionality and citric acid. Part 1 and part 2 can be mixed and a cure takes place through the vinyl group reacting with the tertiary carbon and displacing hydrogen which causes chain extension and crosslinking. The 2-part mixed solution can be cast and warmed (to accelerate the cure) to provide a silicone gel containing citric acid which can then be perforated. Alternatively, other types of silicones that can be used are condensation types, and are usually single component that release a small molecule as the reaction (chain extension / crosslinking) proceeds: alkoxy (releases an alcohol), oxime (releases a ketone) and acetoxy (releases an acid). There is also a reaction type of polymerization / cure for silicones that uses UV and may be a single part format.

### Coating Citric Acid

Additionally or alternatively, in some embodiments citric acid is present as a coating on one or more surfaces of the second layer 210 and/or the third layer 215 and/or any of the optional wicking layers. Thus, in some embodiments, a citric acid-containing polymer solution may be applied to one or more surfaces of the second layer 210 and/or the third layer 215 and/or any optional wicking layers to form a coating comprising citric acid on one or more surfaces of the second layer 210 and/or the third layer 215 and/or optional wicking layers.

In some embodiments, when citric acid is applied as a coating the citric acid may be carried or dispersed into a water-soluble or swollen material, such that when exposed to aqueous media, such as wound fluid, the citric acid is released. The citric acid-containing polymer solution may comprise a water-soluble polymer such as polyvinyl alcohol (PVOH), polyethylene oxide (PEO), polypropylene oxide (PPO), a sodium and/or potassium salt of a carboxylated polymer, a hydroxy and carboxylic acid modified polyacrylic, polyvinyl pyrrolidone (PVP), polyvinyl acetate (PVA), a water-soluble polyurethane, and a copolymer of PVA and PVP. The citric acid-containing polymer solution may contain up to 20% of the water-soluble polymer, for example 1% to 20% w/w or 5% to 15%; and the citric acid-containing polymer solution may contain up to 30% citric acid, for example, 1% to 30% or 5% to 25%.

Alternatively, non-water / alcohol soluble polymers may be formed into dispersions or emulsions, where the continuous phase is water and citric acid has been dissolved in the continuous phase. Examples of non-water / alcohol soluble polymers include a polyester, a polyamide, a polyurethane, a thermoplastic elastomer and an acrylic.

All of the above polymers (except PVOH) may be melt processable (copolymers of PVOH may be melt processable) and the citric acid may be added to the melted polymer in substantially the same manner as a pigment or dye additive.

In further embodiments, the citric acid-containing polymer solution may comprise or further comprise a mixture of collagen and oxidized regenerated cellulose, and optionally an anti-microbial agent such as silver.

The citric acid-containing polymer solution may be covalently bound or non-covalently bound and may be applied to one or more surfaces of the second layer 210 and/or the third layer 215 and/or optional wicking layers by any suitable means such as pattern-coating, deposition-coating or plasma-coating. For example, in some embodiments, the citric acid-containing polymer solution may also contain a photoinitiator and may be applied to a surface of the second layer 210 and/or the third layer 215 and/or optional wicking layers, dried and then exposed to UV light to cure the coating to the surface.

Additionally, in some embodiments, one or more surfaces of the second layer 210 and/or the third layer 215 and/or optional wicking layers may be surface functionalized to covalently bind the citric acid to one or more surfaces of the second layer 210 and/or the third layer 215 and/or the optional wicking layers. As noted above, surface functionalization systems are known, such as the OptoDex@ system commercially available from CSEM Landquart, Landquart, Switzerland. Without being bound by theory, by surface functionalizing the second layer 210 and/or the third layer 215 and/or an optional wicking layer and then applying the citric acid-containing polymer solution, one can modulate the durability of the coating for the presumed wear time of the dressing (e.g., 2, 3, 5, 6, 7, etc... days) so that, if advantageous, the citric acid may be allowed to be mobile.

In further embodiments, the citric acid-containing polymer solution may be simply applied and then dried on a surface of the second layer 210 and/or the third layer 215 and/or the optional wicking layer.

### Assembly of Example Dressings

Additionally, individual components of the dressing 110 in the examples of Figures 2-8 may be bonded or otherwise secured to one another with a solvent or non-solvent adhesive, or with thermal welding, for example, without adversely affecting fluid management. Further, the second layer 210 or the first layer 205 may be coupled to the interior border 250 or the overlay margin 415 of the third layer 215 in any suitable manner, such as with a weld or an adhesive, for example.

The cover 125, the first layer 205, the second layer 210, the third layer 215, or various combinations may be assembled before application or *in situ.* For example, the second layer 210 may be laminated to the first layer 205 in some embodiments. The cover 125 may be disposed over the first layer 205 and coupled to the third layer 215 around the first layer 205 in some embodiments. In some embodiments, one or more layers of the tissue interface 120 may be coextensive. For example, the second layer 210 may be cut flush with the edge of the first layer 205. In some embodiments, the dressing 110 may be provided as a single, composite dressing. For example, the third layer 215 may be coupled to the cover 125 to enclose the first layer 205 and the second layer 210, wherein the third layer 215 may be configured to face a tissue site.

### Example Modes of Treatment

### Dressing Placement

In use, the release liner 260 (if included) may be removed to expose the third layer 215, which can provide a lower surface of the dressing 110 to be placed within, over, on, or otherwise proximate to a tissue site, particularly a surface tissue site and adjacent epidermis. The second layer 210, the third layer 215, or both may be interposed between the first layer 205 and the tissue site, which can substantially reduce or eliminate adverse interaction between the first layer 205 and the tissue site. For example, the third layer 215 may be placed over a surface wound (including edges of the wound) and undamaged epidermis to prevent direct contact with the first layer 205. In some applications, the treatment aperture 230 of the third layer 215 may be positioned adjacent to, proximate to, or covering a tissue site. In some applications, at least some portion of the second layer 210, the fluid restrictions 220, or both may be exposed to a tissue site through the treatment aperture 230, the apertures 235, or both. The periphery 225 of the third layer 215 may be positioned adjacent to or proximate to tissue around or surrounding the tissue site. The third layer 215 may be sufficiently tacky to hold the dressing 110 in position, while also allowing the dressing 110 to be removed or re-positioned without trauma to the tissue site.

Removing the release liner 260 can also expose the adhesive 255, and the cover 125 may be attached to an attachment surface, such as the periphery 225 or other area around the treatment aperture 235 and the first layer 205. The adhesive 255 may also be attached to epidermis peripheral to a tissue site, around the first layer 205 and the second layer 210. For example, the adhesive 255 may be in fluid communication with an attachment surface through the apertures 235 in at least the periphery 225 of the third layer 215. The adhesive 255 may also be in fluid communication with the edges 245 through the apertures 235 exposed at the edges 245.

Once the dressing 110 is in the desired position, the adhesive 255 may be pressed through the apertures 235 to bond the dressing 110 to the attachment surface. The apertures 235 at the edges 245 may permit the adhesive 255 to flow around the edges 245 for enhancing the adhesion of the edges 245 to an attachment surface.

In some embodiments, the apertures 235 may be sized to control the amount of the adhesive 255 exposed through the apertures 235. For a given geometry of the corners 240, the relative sizes of the apertures 235 may be configured to maximize the surface area of the adhesive 255 exposed and in fluid communication through the apertures 235 at the corners 240. For example, the edges 245 may intersect at substantially a right angle, or about 90 degrees, to define the corners 240. In some embodiments, the corners 240 may have a radius of about 10 millimeters. Further, in some embodiments, three of the apertures 235 may be positioned in a triangular configuration at the corners 240 to maximize the exposed surface area for the adhesive 255. In other embodiments, the size and number of the apertures 235 in the corners 240 may be adjusted as necessary, depending on the chosen geometry of the corners 240, to maximize the exposed surface area of the adhesive 255. Further, the apertures 235 at the corners 240 may be fully contained within the third layer 215, substantially precluding fluid communication in a lateral direction exterior to the corners 240. The apertures 235 at the corners 240 being fully contained within the third layer 215 may substantially preclude fluid communication of the adhesive 255 exterior to the corners 240, and may provide improved handling of the dressing 110 during deployment at a tissue site. Further, the exterior of the corners 240 being substantially free of the adhesive 255 may increase the flexibility of the corners 240 to enhance comfort.

In some embodiments, the bond strength of the adhesive 255 may vary based on the configuration of the third layer 215. For example, the bond strength may vary based on the size of the apertures 235. In some examples, the bond strength may be inversely proportional to the size of the apertures 235. Additionally or alternatively, the bond strength may vary in different locations, for example, if the size of the apertures 235 varies. For example, a lower bond strength in combination with larger apertures 235 may provide a bond comparable to a higher bond strength in locations having smaller apertures 235.

The geometry and dimensions of the tissue interface 120, the cover 125, or both may vary to suit a particular application or anatomy. For example, the geometry or dimensions of the tissue interface 120 and the cover 125 may be adapted to provide an effective and reliable seal against challenging anatomical surfaces, such as an elbow or heel, at and around a tissue site. Additionally or alternatively, the dimensions may be modified to increase the surface area for the third layer 215 to enhance the movement and proliferation of epithelial cells at a tissue site and reduce the likelihood of granulation tissue in-growth.

Thus, the dressing 110 can provide a sealed therapeutic environment proximate to a tissue site, substantially isolated from the external environment, and the negative-pressure source 105 can reduce the pressure in the sealed therapeutic environment. The treatment aperture 230 can provide an open area for delivery of negative pressure and passage of wound fluid through the second layer 210 and the first layer 205. The third layer 215 may provide an effective and reliable seal against challenging anatomical surfaces, such as an elbow or heel, at and around a tissue site. Further, the dressing 110 may permit re-application or re-positioning, to correct air leaks caused by creases and other discontinuities in the dressing 110, for example. The ability to rectify leaks may increase the efficacy of the therapy and reduce power consumption in some embodiments.

If not already configured, the dressing interface 270 may be disposed over the aperture 275 and attached to the cover 125. The fluid conductor 265 may be fluidly coupled to the dressing interface 270 and to the negative-pressure source 105.

### Negative Pressure and Instillation Application

Negative pressure applied across the tissue site through the tissue interface 120 in the sealed therapeutic environment can induce macro-strain and micro-strain in the tissue site. Negative pressure can also remove exudate and other fluid from a tissue site, which can be collected in container 115.

The fluid mechanics of using a negative-pressure source to reduce pressure in another component or location, such as within a sealed therapeutic environment, can be mathematically complex. However, the basic principles of fluid mechanics applicable to negative-pressure therapy and instillation are generally well-known to those skilled in the art. In general, exudate and other fluid flow toward lower pressure along a fluid path.

Negative pressure applied through the tissue interface 120 can create a negative pressure differential across the fluid restrictions 220 in the second layer 210, which can open or expand the fluid restrictions 220. For example, in some embodiments in which the fluid restrictions 220 may comprise substantially closed fenestrations through the second layer 210, a pressure gradient across the fenestrations can strain the adjacent material of the second layer 210 and increase the dimensions of the fenestrations to allow liquid movement through them, similar to the operation of a duckbill valve. Opening the fluid restrictions 220 can allow exudate and other liquid movement through the fluid restrictions 220 into the first layer 205. The first layer 205 can provide passage of negative pressure and wound fluid, which can be collected in the container 115. Changes in pressure can also cause the first layer 205 to expand and contract, and the second layer 210, the third layer 215, or both may protect the epidermis from irritation that could be caused by expansion, contraction, or other movement of the first layer 205. For example, in some embodiments, the overlay margin 415 may be disposed between the first layer 205 and epidermis around a tissue site. The second layer 210 and the third layer 215 can also substantially reduce or prevent exposure of a tissue site to the first layer 205, which can inhibit growth of tissue into the first layer 205. For example, the second layer 210 may cover the treatment aperture 230 to prevent direct contact between the first layer 205 and a tissue site.

If the negative-pressure source 105 is removed or turned off, the pressure differential across the fluid restrictions 220 can dissipate, allowing the fluid restrictions 220 to close and prevent exudate or other liquid from returning to the tissue site through the second layer 210.

In some applications, a filler may also be disposed between a tissue site and the third layer 215. For example, if the tissue site is a surface wound, a wound filler may be applied interior to the periwound, and the third layer 215 may be disposed over the periwound and the wound filler. In some embodiments, the filler may be a manifold, such as an open-cell foam. The filler may comprise or consist essentially of the same material as the first layer 205 in some embodiments.

Additionally or alternatively, instillation solution or other fluid may be distributed to the dressing 110, which can increase the pressure in the tissue interface 120. The increased pressure in the tissue interface 120 can create a positive pressure differential across the fluid restrictions 220 in the second layer 210, which can open the fluid restrictions 220 to allow the instillation solution or other fluid to be distributed to the tissue site.

In some embodiments, the controller 130 may receive and process data from one or more sensors, such as the first sensor 135. The controller 130 may also control the operation of one or more components of the therapy system 100 to manage the pressure delivered to the tissue interface 120. In some embodiments, controller 130 may include an input for receiving a desired target pressure and may be programmed for processing data relating to the setting and inputting of the target pressure to be applied to the tissue interface 120. In some example embodiments, the target pressure may be a fixed pressure value set by an operator as the target negative pressure desired for therapy at a tissue site and then provided as input to the controller 130. The target pressure may vary from tissue site to tissue site based on the type of tissue forming a tissue site, the type of injury or wound (if any), the medical condition of the patient, and the preference of the attending physician. After selecting a desired target pressure, the controller 130 can operate the negative-pressure source 105 in one or more control modes based on the target pressure and may receive feedback from one or more sensors to maintain the target pressure at the tissue interface 120.

In some embodiments, the controller 130 may have a continuous pressure mode, in which the negative-pressure source 105 is operated to provide a constant target negative pressure for the duration of treatment or until manually deactivated. Additionally or alternatively, the controller may have an intermittent pressure mode. For example, the controller 130 can operate the negative-pressure source 105 to cycle between a target pressure and atmospheric pressure. For example, the target pressure may be set at a value of 135 mmHg for a specified period of time (e.g., 5 min), followed by a specified period of time (e.g., 2 min) of deactivation. The cycle can be repeated by activating the negative-pressure source 105, which can form a square wave pattern between the target pressure and atmospheric pressure.

In some example embodiments, the increase in negative-pressure from ambient pressure to the target pressure may not be instantaneous. For example, the negative-pressure source 105 and the dressing 110 may have an initial rise time. The initial rise time may vary depending on the type of dressing and therapy equipment being used. For example, the initial rise time for one therapy system may be in a range of about 20-30 mmHg/second and in a range of about 5-10 mmHg/second for another therapy system. If the therapy system 100 is operating in an intermittent mode, the repeating rise time may be a value substantially equal to the initial rise time.

In some example dynamic pressure control modes, the target pressure can vary with time. For example, the target pressure may vary in the form of a triangular waveform, varying between a negative pressure of 50 and 135 mmHg with a rise time set at a rate of +25 mmHg/min. and a descent time set at -25 mmHg/min. In other embodiments of the therapy system 100, the triangular waveform may vary between negative pressure of 25 and 135 mmHg with a rise time set at a rate of +30 mmHg/min and a descent time set at -30 mmHg/min.

In some embodiments, the controller 130 may control or determine a variable target pressure in a dynamic pressure mode, and the variable target pressure may vary between a maximum and minimum pressure value that may be set as an input prescribed by an operator as the range of desired negative pressure. The variable target pressure may also be processed and controlled by the controller 130, which can vary the target pressure according to a predetermined waveform, such as a triangular waveform, a sine waveform, or a saw-tooth waveform. In some embodiments, the waveform may be set by an operator as the predetermined or time-varying negative pressure desired for therapy.

In some embodiments, the controller 130 may receive and process data, such as data related to instillation solution provided to the tissue interface 120. Such data may include the type of instillation solution prescribed by a clinician, the volume of fluid or solution to be instilled to a tissue site ("fill volume"), and the amount of time prescribed for leaving solution at a tissue site ("dwell time") before applying a negative pressure to the tissue site. The fill volume may be, for example, between 10 and 500 mL, and the dwell time may be between one second to 30 minutes. The controller 130 may also control the operation of one or more components of the therapy system 100 to instill solution. For example, the controller 130 may manage fluid distributed from the solution source 145 to the tissue interface 120. In some embodiments, fluid may be instilled to a tissue site by applying a negative pressure from the negative-pressure source 105 to reduce the pressure at the tissue site, drawing solution into the tissue interface 120. In some embodiments, solution may be instilled to a tissue site by applying a positive pressure from the positive-pressure source 150 to move solution from the solution source 145 to the tissue interface 120. Additionally or alternatively, the solution source 145 may be elevated to a height sufficient to allow gravity to move solution into the tissue interface 120.

The controller 130 may also control the fluid dynamics of instillation by providing a continuous flow of solution or an intermittent flow of solution. Negative pressure may be applied to provide either continuous flow or intermittent flow of solution. The application of negative pressure may be implemented to provide a continuous pressure mode of operation to achieve a continuous flow rate of instillation solution through the tissue interface 120, or it may be implemented to provide a dynamic pressure mode of operation to vary the flow rate of instillation solution through the tissue interface 120. Alternatively, the application of negative pressure may be implemented to provide an intermittent mode of operation to allow instillation solution to dwell at the tissue interface 120. In an intermittent mode, a specific fill volume and dwell time may be provided depending, for example, on the type of tissue site being treated and the type of dressing being utilized. After or during instillation of solution, negative-pressure treatment may be applied. The controller 130 may be utilized to select a mode of operation and the duration of the negative pressure treatment before commencing another instillation cycle.

### EXAMPLES

### Example 1 - citric acid coating, covalently bound using UV cure

Dissolve polyethylene oxide in deionized water to form a 20%w/w solution (for example, 200g polyethylene oxide diluted with water to make 1000g). Add 1%w/w and 4% w/w (based on polymer) of UV photoinitiator, such as monoacylphosphineoxide or its sodium / potassium salts, and disperse / dissolve. Add 60g of citric acid to the polymer solution and mix until dissolved; the solution now contains 6% w/w citric acid. The solution has a total solids of 26% w/w and may be suitable for coating low absorbency films, such as a polyurethane film or silicone gel, where the coating thickness or coverage (g/m²) may be used to adjust the concentration of citric acid in relation to the film. For example, a film that is 20g/m², requires a citric acid coverage of 2g/m² (10% of the film), therefore 2/0.06 = 33g of solution over a square meter which equates to the wet thickness of 33 microns. Once coated and dried, expose the film to UVA light (320nm to 500nm) for up to 3 minutes to achieve a covalent cure.

### Example 2 - citric acid coating, covalently bound using OptoDex@ surface functionalization technology

The OptoDex^{®} system commercially available from CSEM Landquart, Landquart, Switzerland is used as a linker molecule to functionalize a surface of the second layer 210, the third layer 215 and/or optional fluid wicking layer. The same citric acid solution can be prepared as in Example 1 and can be covalently bound to functionalized surface of the second layer 210, the third layer 215 and/or optional fluid wicking layer using UV light or localized application of heat. Once activated with UV light or heat, the linker molecule forms covalent bonds between the surface and citric acid solution.

### Example 3 - citric acid coating, non-covalently bound

Prepare a 5% to 20% w/w polymer solution using polyvinyl pyrrolidone (molecular weight [MW] between 40,000 to 400,00) as the carrier, with glycerin up to 10% of the polymer content as a plasticizer, 1% to 10% w/w citric acid, all dissolved in water or water alcohol (ethanol or isopropyl alcohol), for example water:alcohol ratios from 100:0 to 50:50. Add ingredients separately to the solvent mix, then finally mix all the solutions together. A film, such as a polyurethane film, may be dip-coated, roll-coated, knife-coated or spray-coated with the citric acid-solution and dried using forced air at up to 140°C, depending on the softening point of the film.

The systems, apparatuses, and methods described herein may provide significant advantages. For example, some dressings containing citric acid provided herein enable a steady release of citric acid, which can provide extended therapy and substantially reduce or prevent high initial dosing and reduce underutilization and waste of therapeutic agents. The addition of citric acid can provide biofilm disruption to help reduce or eliminate infection especially in long wear dressings. Additionally, some dressings for negative-pressure therapy can require time and skill to be properly sized and applied to achieve a good fit and seal. In contrast, some embodiments of the dressing 110 provide a negative-pressure dressing that is simple to apply, reducing the time to apply and remove. In some embodiments, for example, the dressing 110 may be a fully-integrated negative-pressure therapy dressing that can be applied to a tissue site (including on the periwound) in one step, without being cut to size, while still providing or improving many benefits of other negative-pressure therapy dressings that require sizing. Such benefits may include good manifolding, beneficial granulation, protection of the peripheral tissue from maceration, protection of the tissue site from shedding materials, and a low-trauma and high-seal bond. These characteristics may be particularly advantageous for surface wounds having moderate depth and medium-to-high levels of exudate. Some embodiments of the dressing 110 may remain on the tissue site for at least 5 days, and some embodiments may remain for at least 7 days. Antimicrobial agents in the dressing 110 may extend the usable life of the dressing 110 by reducing or eliminating infection risks that may be associated with extended use, particularly use with infected or highly exuding wounds.

While shown in a few illustrative embodiments, a person having ordinary skill in the art will recognize that the systems, apparatuses, and methods described herein are susceptible to various changes and modifications that fall within the scope of the appended claims. Moreover, descriptions of various alternatives using terms such as "or" do not require mutual exclusivity unless clearly required by the context, and the indefinite articles "a" or "an" do not limit the subject to a single instance unless clearly required by the context. Components may be also be combined or eliminated in various configurations for purposes of sale, manufacture, assembly, or use. For example, in some configurations the dressing 110, the container 115, or both may be eliminated or separated from other components for manufacture or sale. In other example configurations, the controller 130 may also be manufactured, configured, assembled, or sold independently of other components.

## Claims

1. A dressing (110) for treating a tissue site with negative pressure, the dressing (110) comprising:
a sealing layer (215) comprising a treatment area having at least one treatment aperture (230);
a fluid control layer (210) comprising a plurality of reversibly openable fluid restrictions (220) comprising perforations in the fluid control layer (210) aligned with the treatment aperture (230);
a manifold (205) adjacent to the sealing layer (215) and/or the fluid control layer (210);
a cover (125) disposed adjacent the manifold (205) opposite the sealing layer (215) and/or fluid control layer (210); and
wherein citric acid is present as a coating on one or more surfaces of the sealing layer (215) and/or one or more surfaces of the fluid control layer (210), the coating comprising citric acid and a water-soluble and/or water-sensitive polymer, wherein the water-soluble and/or water-sensitive polymer comprises a sodium and/or potassium salt of a carboxylated polymer, a hydroxy and carboxylic acid modified polyacrylic, a water-soluble polyurethane, polyvinyl alcohol (PVOH), polyethylene oxide (PEO), polypropylene oxide (PPO), polyvinyl pyrrolidone (PVP), polyvinyl acetate (PVA), a copolymer of PVA and PVP, or a combination thereof.

2. The dressing (110) of claim 1, wherein the citric acid is covalently bound to one or more surfaces of the sealing layer (215) and/or one or more surfaces of the fluid control layer (210).

3. The dressing (110) of any one of the previous claims, wherein the citric acid is non-covalently bound to one or more surfaces of the sealing layer (215) and/or one or more surfaces of the fluid control layer (210).

4. The dressing (110) of any one of the previous claims, wherein the citric acid is on a surface of the treatment area of the sealing layer (215).

5. The dressing (110) of any one of the previous claims, wherein the sealing layer (215) and/or the fluid control layer (210) comprises citric acid, optionally wherein the sealing layer (215) and/or the fluid control layer (210) comprises about 1% w/w to about 10% w/w amount of citric acid based on the total weight of the respective sealing layer (215) and/or fluid control layer (210).

6. The dressing (110) of any one of the previous claims further comprising a collagen and oxidized regenerated cellulose (ORC) mixture, wherein the collagen and ORC mixture is present as a distinct layer in the dressing (110) and/or present on the surface of the sealing layer (215) and/or the surface of the fluid control layer (210), optionally wherein the citric acid is at least partially encapsulated in the collagen and ORC mixture.

7. The dressing (110) of any one of the previous claims further comprising one or more fluid wicking layers adjacent to the manifold (205).

8. The dressing (110) of any one of the previous claims, wherein the fluid control layer (210) comprises a film of polyurethane, polythene, acrylic, or polyester, optionally wherein the fluid restrictions (220) comprise slits in the film, optionally wherein the slits each have a length of about 2 mm to about 5 mm, optionally wherein the slits each have a length of about 3 mm.

9. The dressing (110) of any one of the previous claims, wherein the sealing layer (215) comprises a polymer gel.

10. The dressing (110) of any one of the previous claims, wherein the sealing layer (215) comprises a silicone gel, a polyurethane gel, or a polythene gel.

11. The dressing (110) of any one of the previous claims, wherein the cover (125) comprises a non-porous film.

12. The dressing (110) of any one of the previous claims, wherein the cover (125) further comprises a pressure-sensitive adhesive.

13. The dressing (110) of any one of the previous claims, wherein the cover (125) is coupled to the sealing layer (215) around the manifold (205).

14. The dressing (110) of any one of the previous claims further comprising an absorbent layer and/or a wicking layer comprising an absorbent.

15. The dressing (110) of any one of the previous claims, wherein:
the manifold (205) has a first edge defining a manifold face adjacent to the fluid control layer (210);
the fluid control layer (210) has a second edge defining a fluid control face adjacent to the manifold face and having a similar shape to the manifold face; and
the manifold face is at least as large as the fluid control face, optionally wherein the fluid control face is larger than the treatment aperture (230).

16. The dressing (110) of any one of the previous claims, wherein at least one of the manifold (205) and the fluid control layer (210) is coupled to a margin around the treatment aperture (230), optionally wherein the margin has a width of about 2 mm to about 3 mm.

17. The dressing (110) of any one of the previous claims, wherein the treatment aperture (230) is complementary to the manifold (205).

18. The dressing (110) of any one of the previous claims, wherein the treatment aperture (230) forms a window around the manifold (205).

19. The dressing (110) of any one of the previous claims, wherein the treatment aperture (230) has a width of about 90 mm to about 110 mm and a length of about 150 mm to about 160 mm.

20. The dressing (110) of any one of the previous claims, wherein the sealing layer (215) has a plurality of perforations, optionally wherein at least some of the perforations are aligned with more than one of the fluid restrictions (220), optionally wherein the perforations are circular and have a diameter of about 7 mm to about 9 mm, optionally wherein the perforations are located around the treatment aperture (230).

21. The dressing (110) of any one of the previous claims, wherein the cover (125), the manifold (205), the fluid control layer (210) and the sealing layer (215) are assembled in a stacked relationship with the cover (125) and the sealing layer (215) enclosing the manifold (205) and the fluid control layer (210).

22. The dressing (110) of any one of the previous claims, wherein the sealing layer (215) has a first surface and a second surface, wherein the second surface is a tissue facing surface and is at least partially coated with an adhesive.

23. The dressing (110) of any one of the previous claims further comprising an adhesive layer coupling the fluid control layer (210) to the sealing layer (215).

## Patentansprüche

1. Ein Verband (110) zum Behandeln einer Gewebestelle mit Unterdruck, der Verband (110) aufweisend:
eine Abdichtungsschicht (215), aufweisend einen Behandlungsbereich, der mindestens eine Behandlungsöffnung (230) hat;
eine Fluidkontrollschicht (210), aufweisend eine Mehrzahl von reversibel öffenbaren Fluidbeschränkungen (220), aufweisend Perforationen in der Fluidkontrollschicht (210), die mit der Behandlungsöffnung (230) ausgerichtet sind;
einen Verteiler (205) angrenzend an die Abdichtungsschicht (215) und/oder die Fluidkontrollschicht (210);
eine Abdeckung (125), die angrenzend an den Verteiler (205) gegenüber der Abdichtungsschicht (215) und/oder der Fluidkontrollschicht (210) angeordnet ist; und
wobei Citronensäure als eine Beschichtung an einer oder mehreren Oberflächen der Abdichtungsschicht (215) und/oder einer oder mehreren Oberflächen der Fluidkontrollschicht (210) vorhanden ist, die Beschichtung aufweisend Citronensäure und ein wasserlösliches und/oder wasserempfindliches Polymer, wobei das wasserlösliche und/oder wasserempfindliche Polymer ein Natrium- und/oder Kaliumsalz eines carboxylierten Polymers, ein hydroxy- und carbonsäuremodifiziertes Polyacryl, ein wasserlösliches Polyurethan, Polyvinylalkohol (PVOH), Polyethylenoxid (PEO), Polypropylenoxid (PPO), Polyvinylpyrrolidon (PVP), Polyvinylacetat (PVA), ein Copolymer aus PVA und PVP oder eine Kombination davon aufweist.

2. Der Verband (110) nach Anspruch 1, wobei die Citronensäure an eine oder mehrere Oberflächen der Abdichtungsschicht (215) und/oder eine oder mehrere Oberflächen der Fluidkontrollschicht (210) kovalent gebunden ist.

3. Der Verband (110) nach einem der vorstehenden Ansprüche, wobei die Citronensäure an eine oder mehrere Oberflächen der Abdichtungsschicht (215) und/oder eine oder mehrere Oberflächen der Fluidkontrollschicht (210) nicht kovalent gebunden ist.

4. Der Verband (110) nach einem der vorstehenden Ansprüche, wobei sich die Citronensäure an einer Oberfläche des Behandlungsbereichs der Abdichtungsschicht (215) befindet.

5. Der Verband (110) nach einem der vorstehenden Ansprüche, wobei die Abdichtungsschicht (215) und/oder die Fluidkontrollschicht (210) Citronensäure aufweist, optional wobei die Abdichtungsschicht (215) und/oder die Fluidkontrollschicht (210) zu etwa 1 Gew.-% bis etwa 10 Gew.-% eine Menge Citronensäure, basierend auf dem Gesamtgewicht der jeweiligen Abdichtungsschicht (215) und/oder Fluidkontrollschicht (210), aufweist.

6. Der Verband (110) nach einem der vorstehenden Ansprüche, ferner aufweisend eine Mischung aus Collagen und oxidierter regenerierter Cellulose (ORC), wobei die Collagen- und ORC-Mischung als eine eindeutige Schicht in dem Verband (110) vorhanden und/oder an der Oberfläche der Abdichtungsschicht (215) und/oder der Oberfläche der Fluidkontrollschicht (210) vorhanden ist, optional wobei die Citronensäure in der Collagen- und ORC-Mischung mindestens teilweise eingekapselt ist.

7. Der Verband (110) nach einem der vorstehenden Ansprüche, ferner aufweisend eine oder mehrere Fluiddochtschichten angrenzend an den Verteiler (205).

8. Der Verband (110) nach einem der vorstehenden Ansprüche, wobei die Fluidkontrollschicht (210) eine Folie aus Polyurethan, Polyethylen, Acryl oder Polyester aufweist, optional wobei die Fluidbeschränkungen (220) Schlitze in der Folie aufweisen, optional wobei die Schlitze je eine Länge von etwa 2 mm bis etwa 5 mm haben, optional wobei die Schlitze je eine Länge von etwa 3 mm haben.

9. Der Verband (110) nach einem der vorstehenden Ansprüche, wobei die Abdichtungsschicht (215) ein Polymergel aufweist.

10. Der Verband (110) nach einem der vorstehenden Ansprüche, wobei die Abdichtungsschicht (215) ein Silicongel, ein Polyurethangel oder ein Polyethylengel aufweist.

11. Der Verband (110) nach einem der vorstehenden Ansprüche, wobei die Abdeckung (125) eine nicht poröse Folie aufweist.

12. Der Verband (110) nach einem der vorstehenden Ansprüche, wobei die Abdeckung (125) ferner einen Haftklebstoff aufweist.

13. Der Verband (110) nach einem der vorstehenden Ansprüche, wobei die Abdeckung (125) mit der Abdichtungsschicht (215) um den Verteiler (205) herum gekoppelt ist.

14. Der Verband (110) nach einem der vorstehenden Ansprüche, ferner aufweisend eine Absorptionsschicht und/oder eine Dochtschicht, aufweisend ein Absorptionsm ittel.

15. Der Verband (110) nach einem der vorstehenden Ansprüche, wobei:
der Verteiler (205) eine erste Kante hat, die eine Verteilerfläche angrenzend an die Fluidkontrollschicht (210) definiert;
die Fluidkontrollschicht (210) eine zweite Kante hat, die eine Fluidkontrollfläche angrenzend an die Verteilerfläche definiert und eine ähnliche Form wie die Verteilerfläche hat; und
die Verteilerfläche mindestens so groß wie die Fluidkontrollfläche ist, optional wobei die Fluidkontrollfläche größer als die Behandlungsöffnung (230) ist.

16. Der Verband (110) nach einem der vorstehenden Ansprüche, wobei mindestens eines von dem Verteiler (205) und der Fluidkontrollschicht (210) mit einem Rand um die Behandlungsöffnung (230) herum gekoppelt ist, optional wobei der Rand eine Breite von etwa 2 mm bis etwa 3 mm hat.

17. Der Verband (110) nach einem der vorstehenden Ansprüche, wobei die Behandlungsöffnung (230) komplementär zu dem Verteiler (205) ist.

18. Der Verband (110) nach einem der vorstehenden Ansprüche, wobei die Behandlungsöffnung (230) ein Fenster um den Verteiler (205) herum ausbildet.

19. Der Verband (110) nach einem der vorstehenden Ansprüche, wobei die Behandlungsöffnung (230) eine Breite von etwa 90 mm bis etwa 110 mm und eine Länge von etwa 150 mm bis etwa 160 mm hat.

20. Der Verband (110) nach einem der vorstehenden Ansprüche, wobei die Abdichtungsschicht (215) eine Mehrzahl von Perforationen hat, optional wobei mindestens einige der Perforationen mit mehr als einer der Fluidbeschränkungen (220) ausgerichtet sind, optional wobei die Perforationen kreisförmig sind und einen Durchmesser von etwa 7 mm bis etwa 9 mm haben, optional wobei die Perforationen um die Behandlungsöffnung (230) herum gelegen sind.

21. Der Verband (110) nach einem der vorstehenden Ansprüche, wobei die Abdeckung (125), der Verteiler (205), die Fluidkontrollschicht (210) und die Abdichtungsschicht (215) in einer gestapelten Beziehung zusammengesetzt sind, wobei die Abdeckung (125) und die Abdichtungsschicht (215) den Verteiler (205) und die Fluidkontrollschicht (210) umschließen.

22. Der Verband (110) nach einem der vorstehenden Ansprüche, wobei die Abdichtungsschicht (215) eine erste Oberfläche und eine zweite Oberfläche hat, wobei die zweite Oberfläche eine einem Gewebe zugewandte Oberfläche ist und mindestens teilweise mit einem Klebstoff beschichtet ist.

23. Der Verband (110) nach einem der vorstehenden Ansprüche, ferner aufweisend eine Klebstoffschicht, die die Fluidkontrollschicht (210) mit der Abdichtungsschicht (215) koppelt.

## Revendications

1. Pansement (110) destiné au traitement d'un site tissulaire avec une pression négative, le pansement (110) comprenant :
une couche d'étanchéité (215) comprenant une zone de traitement ayant au moins une ouverture de traitement (230) ;
une couche de régulation de fluide (210) comprenant une pluralité de restrictions de fluide pouvant être ouvertes de manière réversible (220) comprenant des perforations dans la couche de régulation de fluide (210) alignées avec l'ouverture de traitement (230) ;
un collecteur (205) adjacent à la couche d'étanchéité (215) et/ou à la couche de régulation de fluide (210) ;
un recouvrement (125) disposé adjacent au collecteur (205) opposé à la couche d'étanchéité (215) et/ou à la couche de régulation de fluide (210) ; et
dans lequel l'acide citrique est présent en tant que revêtement sur une ou plusieurs surfaces de la couche d'étanchéité (215) et/ou une ou plusieurs surfaces de la couche de régulation de fluide (210), le revêtement comprenant de l'acide citrique et un polymère hydrosoluble et/ou sensible à l'eau, dans lequel le polymère hydrosoluble et/ou sensible à l'eau comprend un sel de sodium et/ou de potassium d'un polymère carboxylé, un polyacrylique modifié par un hydroxy et un acide carboxylique, un polyuréthane hydrosoluble, un alcool polyvinylique (PVOH), un oxyde de polyéthylène (POE), un oxyde de polypropylène (PPO), un polyvinyl pyrrolidone (PVP), un acétate de polyvinyle (PVA), un copolymère de PVA et de PVP, ou une combinaison de ceux-ci.

2. Pansement (110) selon la revendication 1, dans lequel l'acide citrique est lié de manière covalente à une ou plusieurs surfaces de la couche d'étanchéité (215) et/ou à une ou plusieurs surfaces de la couche de régulation de fluide (210).

3. Pansement (110) selon l'une quelconque des revendications précédentes, dans lequel l'acide citrique est lié de manière non covalente à une ou plusieurs surfaces de la couche d'étanchéité (215) et/ou à une ou plusieurs surfaces de la couche de régulation de fluide (210).

4. Pansement (110) selon l'une quelconque des revendications précédentes, dans lequel l'acide citrique est sur une surface de la zone de traitement de la couche d'étanchéité (215).

5. Pansement (110) selon l'une quelconque des revendications précédentes, dans lequel la couche d'étanchéité (215) et/ou la couche de régulation de fluide (210) comprennent de l'acide citrique, facultativement dans lequel la couche d'étanchéité (215) et/ou la couche de régulation de fluide (210) comprennent une quantité d'acide citrique d'environ 1 % p/p à environ 10 % p/p sur la base du poids total de la couche d'étanchéité (215) respective et/ou de la couche de régulation de fluide (210).

6. Pansement (110) selon l'une quelconque des revendications précédentes comprenant en outre un mélange de collagène et de cellulose régénérée oxydé (ORC), dans lequel le mélange de collagène et de cellulose est présent en tant que couche distincte dans le pansement (110) et/ou présent sur la surface de la couche d'étanchéité (215) et/ou sur la surface de la couche de régulation de fluide (210), facultativement dans lequel l'acide citrique est au moins partiellement encapsulé dans le mélange de collagène et d'ORC.

7. Pansement (110) selon l'une quelconque des revendications précédentes, comprenant en outre une ou plusieurs couches à effet de mèche de fluide adjacentes au collecteur (205).

8. Pansement (110) selon l'une quelconque des revendications précédentes, dans lequel la couche de régulation de fluide (210) comprend un film de polyuréthane, polythène, acrylique ou polyester, facultativement dans lequel les restrictions de fluide (220) comprennent des fentes dans le film, facultativement dans lequel les fentes ont chacune une longueur d'environ 2 mm à environ 5 mm, facultativement dans lequel les fentes ont chacune une longueur d'environ 3 mm.

9. Pansement (110) selon l'une quelconque des revendications précédentes, dans lequel la couche d'étanchéité (215) comprend gel de polymère ;

10. Pansement (110) selon l'une quelconque des revendications précédentes, dans lequel la couche d'étanchéité (215) comprend un gel de silicone, un gel de polyuréthane ou un gel de polythène.

11. Pansement (110) selon l'une quelconque des revendications précédentes, dans lequel le recouvrement (125) est poreux ou non poreux.

12. Pansement (110) selon l'une quelconque des revendications précédentes, dans lequel le recouvrement (125) comprend en outre un adhésif sensible à la pression.

13. Pansement (110) selon l'une quelconque des revendications précédentes, dans lequel le recouvrement (125) est accouplé à la couche d'étanchéité (215) autour du collecteur (205).

14. Pansement (110) selon l'une quelconque des revendications précédentes, comprenant en outre une couche absorbante et/ou une couche à effet de mèche comprenant un absorbant.

15. Pansement (110) selon l'une quelconque des revendications précédentes, dans lequel :
le collecteur (205) a un premier bord définissant une face de collecteur adjacente à la couche de régulation de fluide (210) ;
la couche de régulation de fluide (210) a un second bord définissant une face de régulation de fluide adjacente à la face de collecteur et ayant une forme similaire à la face de collecteur ; et
la face de collecteur est au moins aussi grande que la face de régulation de fluide, facultativement dans lequel la face de régulation de fluide est plus grande que l'ouverture de traitement (230).

16. Pansement (110) selon l'une quelconque des revendications précédentes, dans lequel au moins un parmi le collecteur (205) et la couche de régulation de fluide (210) est accouplé à une marge autour de l'ouverture de traitement (230), facultativement dans lequel la marge a une largeur d'environ 2 mm à environ 3 mm.

17. Pansement (110) selon l'une quelconque des revendications précédentes, dans lequel l'ouverture de traitement (230) est complémentaire au collecteur (205).

18. Pansement (110) selon l'une quelconque des revendications précédentes, dans lequel l'ouverture de traitement (230) forme une fenêtre autour du collecteur (205).

19. Pansement (110) selon l'une quelconque des revendications précédentes, dans lequel le collecteur (230) a une épaisseur d'environ 90 mm à environ 110 mm, et une longueur d'environ 150 mm à environ 160 mm.

20. Pansement (110) selon l'une quelconque des revendications précédentes, dans lequel la couche d'étanchéité (215) a une pluralité de perforations, facultativement dans lequel au moins certaines des perforations sont alignées avec plus d'une des restrictions de fluide (220), facultativement dans lequel les perforations sont circulaires et ont un diamètre d'environ 7 mm à environ 9 mm, facultativement dans lequel les perforations sont situées autour de l'ouverture de traitement (230).

21. Pansement (110) selon l'une quelconque des revendications précédentes, dans lequel le recouvrement (125), le collecteur (205), la couche de régulation de fluide (210) et la couche d'étanchéité (215) sont assemblés dans une relation empilée avec le recouvrement (125) et la couche d'étanchéité (215) renfermant le collecteur (205) et la couche de régulation de fluide (210).

22. Pansement (110) selon l'une quelconque des revendications précédentes, dans lequel la couche d'étanchéité (215) a une première surface et une seconde surface, dans lequel la seconde surface est une surface faisant face au tissu et est au moins partiellement revêtue d'un adhésif.

23. Pansement (110) selon l'une quelconque des revendications précédentes, comprenant en outre une couche adhésive accouplant la couche de régulation de fluide (210) à la couche d'étanchéité (215).
